# EUROPEAN PATENT APPLICATION

(11) **EP 4 529 891 A1**
(43) Date of publication of application: **02.04.2025**
(21) Application number: 24200963.7
(22) Date of filing: 18.09.2024
(51) Int. Cl.: A61F 2/46, A61F 2/40, A61F 2/30, A61B 17/16, A61B 17/17

(54) **HUMERAL RECONSTRUCTION SYSTEM**

(30) Priority: 28.09.2023 US 202363586016 P
(71) Applicant: Howmedica Osteonics Corp., Mahwah, NJ 07430 (US)
(72) Inventor: SPLIETH, Roy Philip, Central Valley, NY, 10917 (US); NAYAK, Amith, Great Meadows, NJ, 07838 (US); ANDREAS, Anthony, Columbia City, IN, 46725 (US); SAPIO, Daniel, Mohegan Lake, NY, 10547 (US); BARILE, Gennaro A., Secaucus, NJ, 07094 (US); PARKER, Brad, Warsaw, IN, 46582 (US); KARTHOLL, Matt, Fort Wayne, IN, 46804 (US); GEELS, Travis, Fort Wayne, IN, 46825 (US); YADAV, Rajan, 110078 Delhi (IN); VERMA, Shashank, 282006 Uttar Pradesh (IN); JAGTAP, Vishal Dilip, 400604 Maharashtra (IN); GERA, Sahil Kumar, 110024 Delhi (IN); MUJAWAR, Arifmohamad Hamaju, 416301 Maharashtra (IN)
(74) Representative: Röthinger, Rainer

(57) **Abstract**

A humeral prosthesis (100) for replacing a proximal humerus includes a prosthesis body (110) which includes a lateral surface (111), a medial surface (114), an anterior surface (113a), a posterior surface (113b), and a first superior surface (115). The lateral surface has a spherical curvature. The anterior and posterior surfaces are each planar and each intersect the first superior surface, the lateral surface, and the medial surface. A first bore (121) extends through the first superior surface and into the prosthesis body. The prosthesis body also includes a plurality of suture openings (124a, 124b).

## Description

### CROSS-REFERENCE TO RELATED APPLICATIONS

This application claims the benefit of U.S. Provisional Application No. 63/586,016, filed on September 28, 2023, the disclosure of which is hereby incorporated by reference.

### BACKGROUND OF THE INVENTION

Over time and through repeated use, bones and joints can become damaged or worn. For example, repetitive strain on bones and joints (*e.g.,* through athletic activity), traumatic events, and certain diseases (*e.g.,* arthritis) can cause the cartilage in joint areas, for example, which normally provides a cushioning effect, to wear down. When the cartilage wears down, fluid can accumulate in the joint areas, resulting in pain, stiffness, and decreased mobility. The same can happen in cases where tendons in a joint become lax or soft tissues in (or adjacent to) the joint become damaged or worn.

Arthroplasty procedures can be used to repair such damaged joints. During a typical arthroplasty procedure, an arthritic or otherwise dysfunctional joint can be remodeled or realigned. A prosthesis or prostheses can be implanted to repair the damaged region(s). Arthroplasty procedures may take place in any of a number of different regions of the body, such as the knees, hips, shoulders, or elbows, for example. One type of arthroplasty procedure is shoulder arthroplasty, in which a damaged shoulder joint may be replaced with one or more prosthetic implants. The shoulder joint may have been damaged by, for example, arthritis (*e.g.,* severe osteoarthritis or degenerative arthritis), trauma, or a destructive joint disease.

A typical anatomical shoulder joint replacement attempts to mimic anatomic conditions. For example, a humeral stem and a humeral head replacement are attached to the humerus of the arm and replace the humeral side of a shoulder joint that is arthritic, has suffered trauma or otherwise requires replacement to improve the condition of the patient. The humeral head replacement can articulate with the native glenoid socket or with an opposing prosthetic glenoid implant.

For more severe cases, a reverse reconstruction can be performed, which includes reversing the kinematics of the shoulder joint. A reverse shoulder prosthesis can be provided by securing a semi-spherical device (sometimes called a glenosphere) to the glenoid and implanting a humeral stem with a socket or cup capable of receiving the glenosphere.

Before implanting the humeral implant, it may be desirable to trial the humeral implant to determine the appropriate length of the stem, the appropriate inclination angle of the articulating head, and/or the size of the articulating head, or other characteristics of the implant. The trial humeral implant can be assembled and then inserted into the humerus. Afterward, the entire trial implant can be removed, and the definitive humeral implant can be chosen and implanted in the bone. In order to determine the desired height for the prosthesis, it may be desirable to have tools to help measure the height between the proximal humeral resection and the center of the glenoid, even before beginning to assemble the trial prosthesis.

### BRIEF SUMMARY OF THE INVENTION

In one aspect of the present disclosure, a humeral prosthesis for replacing a proximal humerus may include a prosthesis body that that may include a lateral surface, a medial surface, an anterior surface, a posterior surface, and a first superior surface. The lateral surface may have a spherical curvature. The anterior and posterior surfaces may each be planar and may each intersect the first superior surface, the lateral surface, and the medial surface. A first bore may extend through the first superior surface and into the prosthesis body. The prosthesis body may also include plurality of suture openings.

Additionally, the plurality of suture openings may include a first suture opening and a second suture opening. The first suture opening may define a first suture opening axis, and the second suture opening may define a second suture opening axis. The first axis and the second axis may intersect at an angle Θ. The angle Θ may be an oblique angle. As an example, angle Θ may be 20 to 60 degrees.

Also, the anterior and posterior surfaces may taper inwardly in a superior to inferior direction and a lateral to medial direction. The anterior and posterior surfaces may each include a porous patch. The porous patch of each of the anterior and posterior surfaces may be surrounded by a nonporous portion. Additionally, the porous patch may be one of flush with the nonporous portion and recessed relative to the nonporous portion.

Furthermore, the anterior surface may intersect the lateral surface at a first rounded edge, and the posterior surface may intersect the lateral surface at a second rounded edge. The medial surface may be curved in at least one plane and may intersects the first superior surface.

Continuing with this aspect, the body may further include a second superior surface that may intersect the first superior surface and the lateral surface. The first and second superior surfaces may each be planar. Also, the first superior surface may be obliquely angled relative to a longitudinal axis of the body, and the second superior surface may be oriented perpendicular to the longitudinal axis of the body. Additionally, the body may further include a second bore extending through the first superior surface and entirely through the body. The first and second bore may intersect. The first bore and the second bore may each be smooth bores. Alternatively, the first bore may include a plurality of scallops arrayed about a central axis of the first bore. Each scallop may be rotationally offset relative to an adjacent scallop by an angle α. The angle α may be 5 to 45 degrees, as an example. The body may further include a distal surface, and the distal surface may be planar. The body may further include a tapered post that may extend from the distal surface. Still further, the body may include a pair of inner legs and a pair of outer legs. Each outer leg may be offset from and parallel to a corresponding inner leg. The slot may extend entirely through each outer leg in a direction toward the inner legs.

The prosthesis may further include a spacer. The spacer may have a first porous ring and first and second spacer suture openings. The first spacer suture opening may be disposed superior and adjacent to the first porous ring. Thee second spacer suture opening may be disposed inferior and adjacent to the first porous ring. The first spacer suture opening may define a first spacer suture opening axis, the second spacer suture opening may define a second spacer suture opening axis, and the first and second spacer suture opening axes may extend parallel to each other. The spacer may include a second porous ring and third and fourth spacer suture openings. The third spacer suture opening may be disposed superior and adjacent to the second porous ring. The fourth spacer suture opening may be disposed inferior and adjacent to the second porous ring. The third spacer suture opening may define a third spacer suture opening axis, the fourth spacer suture opening may define a fourth spacer suture opening axis, and the first and second spacer suture opening axes may extend perpendicular to the third and fourth spacer suture opening axes.

The prosthesis may also include a stem component that may have an adapter and a stem that may extend distally from the adapter. The stem may include a first stem portion and a second stem portion. The first and second stem portions may be moveable in a radial direction between a first position and a second position.

In another aspect of the present disclosure, a system for replacing a proximal humerus may include a prosthesis body that may include a first superior surface, an anterior surface, a posterior surface, a lateral surface, a medial surface, and a first bore extending through the first superior surface. The first bore may have a plurality of scallops arrayed about a central axis of the first bore. The system may also include an articular trial component that may have a trial body and a boss that may extend from the trial body. The trial body may have an articular surface disposed thereon. The boss may be configured to be received within the first bore, may have a protrusion that may extend radially outwardly therefrom, and may be configured to engage one of the scallops.

Additionally, the trial body may include a central axis, and the boss may define a boss axis. The central axis may be offset from the central axis, and the prosthesis body may include a midline plane bisecting the prosthesis body and may extend in a superior-inferior direction and a medial-lateral direction. The articular trial component may have a first eccentricity configuration relative to the prosthesis body in which the protrusion may engage a first scallop and the central axis of the trial body and the boss axis may be coplanar with the midline plane. The articular trial component may also have a second eccentricity configuration relative to the prosthesis body in which the protrusion may engage a second scallop, the boss axis may be coplanar with the midline plane, and the central axis of the trial body may be offset in one of an anterior and posterior direction relative to the midline plane. The trial body may include a central opening that may be coaxial with the central axis of the trial body, and a through bore may extend through the trial body and boss. The through-bore may be coaxial with the boss axis.

Furthermore, the articular surface may be a concave articular surface. Alternatively, the articular surface may be a convex articular surface. Also, the prosthesis body may have a second bore extending entirely therethrough. The system may also include a trial adapter disposed within the second bore and may have a flange that may partially define the first bore of the prosthesis body. The scallops may be disposed within the flange.

In a further aspect of the present disclosure, a bone clamp assembly for resecting a proximal humerus may include a first member that may have a first handle and a first jaw. The clamp assembly may also include a second member that may be pivotably connected to the first handle and may have a second handle and a second jaw. Additionally, the assembly may include a resection guide that may be connectable to the second jaw. The resection guide may have at least one of a captured guide slot and an uncaptured guide surface.

Also, the second jaw may include a first opening that may extend therein. The resection guide may include a first post that may extend therefrom and may be configured to be received within the first opening. The resection guide may include a second post, and the second jaw may include a moveable plunger that may extend from a free end thereof and may have a captured slot extending therein. The second post may be receivable within the captured slot. The second jaw may a spring, and the plunger may be moveable between a first position and a second position. The spring may bias the plunger toward the first position. The plunger may include a cam surface, the first post of the resection guide may define a rotational axis about which resection guide rotates, and the second post may be configured to engage the cam surface when the resection guide is rotated about the rotational axis in a first direction which may cause the plunger to move from the first position to the second position. The plunger may include a protrusion that may extend into the captured slot, and the second post may have a notch configured to receive the protrusion when the second post is positioned within the captured slot.

The first jaw may have a first row of teeth that may extend along an arcuate path, and the second jaw may include a first row of teeth forming a "V"-shape configuration. The second jaw may include a second row of teeth that may form a "V"-shape configuration. The bone clamp may define a first axis that may extend along a length of the handles and located equidistant therebetween, and a second axis may extend along a length of jaws and located equidistant therebetween. The first may intersect the second axis at an angle β. The angle β may be 120 to 140 degrees, as an example. The angle β may be 130 degrees, as another example.

In a still further aspect of the present disclosure, a method for replacing a proximal portion of a humerus may include clamping first and second jaws of a clamp assembly onto a humeral shaft; guiding a bone saw through a captured guide slot or along a non-captured guide surface of a resection guide of the clamp assembly to resect the proximal portion of the humerus; and inserting a stem component of a humeral prosthesis into the shaft of the humerus. The humeral prosthesis may include a prosthesis body and one or more spacers connected to the prosthesis body and stem component.

Additionally, the clamping step may include engaging the first jaw to a posterior aspect of the humeral shaft and the second jaw to an anterior aspect of the humeral shaft. The method may also include connecting the resection guide to the second jaw by inserting a first post into a first opening in the second jaw and rotating the resection guide in a first direction until a second post of the resection guide is received within a slot of the second jaw. The method may also include connecting an articular trial component in a first eccentric configuration in which a boss of the articular trial component is received within a first bore of the prosthesis body and a protrusion of the boss engages a first scallop at least partially defining the first bore. The articular trial component may have a trial body and an articular surface disposed on the trial body. Also, the method may include rotating the articular trial component to a second eccentric configuration in which the protrusion of the boss may engage a second scallop at least partially defining the first bore. Furthermore, the method may include connecting the one or more spacers to the stem component and the prosthesis body via a taper lock connection.

Still further, the method may include connecting the one or more spacers to the stem component and the prosthesis body via a snap-fit connection. Additionally, the method may include passing suture through a first and second suture opening in the one or more spacers and through soft tissue, and compressing the soft tissue against a porous structure between the first and second suture openings via the suture. Also, the method may include connecting an anatomy tracker to the bone clamp, and registering the humerus in a computer aided surgical system using the anatomy tracker.

### BRIEF DESCRIPTION OF THE DRAWINGS

FIG. 1A is a schematic view of a human shoulder joint showing the bones thereof.
FIG. 1B is a schematic view of a humerus of the human shoulder joint of FIG. 1A.
FIG. 2A is a is a perspective view of an exemplary humeral prosthesis according to an embodiment of the present disclosure as implanted into the humerus of FIG. 1A and including an exemplary a reverse articular insert and an exemplary reverse insert tray.
FIGS. 2B is a front elevational view of an exemplary proximal body that may be implemented in the humeral prosthesis of FIG. 2A and in accordance with an embodiment of the present disclosure.
FIGS. 2C and 2D are perspective views of the proximal body of FIG. 2B.
FIG. 2E is a perspective view of an exemplary proximal body that may be implemented in the humeral prosthesis of FIG. 2A and in accordance with another embodiment of the present disclosure.
FIG. 2F is a front elevational view of an exemplary kit of proximal bodies like that of FIG. 2B and in accordance with an embodiment of the present disclosure.
FIG. 2G is a perspective view of an exemplary stem component that may be implemented in the humeral prosthesis of FIG. 2A and in accordance with an embodiment of the present disclosure.
FIG. 2H is a cross-sectional view of the stem component of FIG. 2G taken at a midline thereof.
FIG. 2I is a perspective view of an exemplary implant spacer that may be implemented in the humeral prosthesis of FIG. 2A and in accordance with an embodiment of the disclosure.
FIG. 2J is a front elevational view of the implant spacer of FIG. 2I.
FIG. 2K is a front elevational view of an exemplary kit of implant spacers like that of FIG. 2I.
FIG. 2L is an exploded view of an exemplary humeral prosthesis like that of FIG. 2A and in accordance with another embodiment of the present disclosure.
FIG. 2M is an elevational view of the humeral prosthesis of FIG. 2A as assembled.
FIG. 2N is a cross-sectional view of the humeral prosthesis of FIG. 2L taken at a midline thereof.
FIG. 3A is a perspective view of an exemplary proximal body trialing adapter in accordance with an embodiment of the present disclosure.
FIG. 3B is a side elevational view of the of the proximal body trialing adapter of FIG. 3A.
FIG. 3C is a partial cross-sectional perspective view of the proximal body trialing adapter within a prosthesis proximal body and taken along a midline thereof.
FIG. 4A is a perspective view of an exemplary reverse trial insert in accordance with to an embodiment of the present disclosure.
FIG. 4B is a front view of the reverse trial insert of FIG. 4A.
FIG. 5A is a perspective view of an exemplary trial tray in accordance with an embodiment of the present disclosure.
FIG. 5B is another perspective view of the trial tray of FIG. 5A.
FIG. 5C is a partial perspective view of a boss of the trial tray of FIG. 5A.
FIG. 5D is a cutaway view of the trial tray of FIG. 5A as assembled with the proximal body trialing adapter.
FIGS. 5E-5G are perspective views of the trial tray as assembled to an implant proximal body in respective first, second, and third configurations.
FIG. 6A is a top perspective view of an exemplary humeral head trial in accordance with an embodiment of the present disclosure.
FIG. 6B is a bottom perspective view of the humeral head trial of FIG. 6A.
FIG. 7 is a perspective view of an exemplary humeral head coupler in accordance with an embodiment of the present disclosure.
FIGS. 8A and 8B are a perspective views of an exemplary humeral head coupler trial in accordance with an embodiment of the present disclosure.
FIG. 9A is a is a perspective view of an exemplary humeral trial prosthesis according to an embodiment of the present disclosure as implanted into the humerus of FIG. 1A and as assembled with the trial tray of FIG. 5A and trial insert of FIG. 4A.
FIGS. 9B and 9C are perspective views of an exemplary proximal trial body that may be implemented in the humeral prosthesis of FIG. 2A and in accordance with an embodiment of the present disclosure.
FIG. 9D is a partial side view of the trial proximal body of FIGS. 9B and 9C.
FIG. 9E is a partial perspective view of an exemplary proximal trial body in accordance with another embodiment of the present disclosure.
FIGS. 9F-9H are perspective views of an exemplary trial spacer that may be implemented in the humeral trial prosthesis of FIG. 9A and in accordance with an embodiment of the present disclosure.
FIG. 9I a front elevational view of an exemplary kit of trial spacers like that of FIG. 9F.
FIG. 9J is an exploded cross-sectional view of a pair of trial spacers from the kit of FIG. 9I.
FIG. 10A is perspective view of an exemplary expandable trial stem that may be implemented in the humeral trial prosthesis of FIG. 9A and in accordance with an embodiment of the present disclosure.
FIG. 10B is an elevational view of an expansion bolt of the expandable trial stem of FIG. 10A.
FIG. 11A is a perspective view of an exemplary taper protector insert that may be implemented in the humeral trial prosthesis of FIG. 9A and in accordance with an embodiment of the present disclosure.
FIG. 11B is a partial exploded view of the taper protector insert as assembled with the stem component of FIG. 2G and in conjunction with the trial spacer of FIG. 9F.
FIG. 12A is a perspective view of an exemplary bone clamp assembly in accordance with an embodiment of the present disclosure and as overlayed by a pair of intersecting axes.
FIG. 12B is a schematic view of the pair of intersecting axes of FIG. 12A.
FIG. 12C is an exploded view of the bone clamp assembly of FIG. 12A.
FIGS. 12D and 12E are partial views of the bone clamp assembly of FIG. 12A with a resection guide thereof in respective first and second configurations.
FIG. 12F is a partial perspective view of the bone clamp assembly of FIG. 12A.
FIG. 12G is a partial perspective view of the bone clamp assembly of FIG. 12A as attached to the humerus of FIG. 1B and in conjunction with an oscillating bone saw.
FIG. 12H is a partial perspective view of an exemplary bone clamp assembly in accordance with another embodiment of the present disclosure.
FIG. 12I is a top view of the bone clamp assembly of FIG. 12H as attached to the humerus of FIG. 1B.
FIG. 13 is a perspective view of an exemplary sounder in accordance with an embodiment of the present disclosure.
FIG. 14A is a perspective view of an exemplary reamer planar in accordance with an embodiment of the present disclosure.
FIG. 14B is a partial perspective view of the reamer planar of FIG. 14A.
FIG. 15A is an elevational view of an exemplary handle in accordance with an embodiment of the present disclosure.
FIG. 15B is a cross-sectional view of the handle of FIG. 15A taken at a midline thereof.
FIG. 15C illustrates the handle of FIG. 15A just prior to engagement with the stem component of FIG. 2G.
FIG. 15D illustrates the handle of FIG. 15A just after engagement with the stem component of FIG. 2G and while in an unlocked position.
FIG. 15E illustrates the handle of FIG. 15A just after engagement with the implant stem and after transitioning to a locked position.
FIG. 16A is a front elevational view of an exemplary height measurement gauge in accordance with an embodiment of the present disclosure.
FIG. 16B is a cross-sectional view of a scale of the height measurement gauge of FIG. 16A taken along a line B-B thereof.
FIG. 16C is a perspective view of the height measurement gauge of FIG. 16A without a pointer member thereof.
FIG. 16D is an enlarged view of the clamp of the height measurement gauge of FIG. 16A.
FIG. 17A is a perspective view of an exemplary impaction stand in accordance with an embodiment of the present disclosure.
FIG. 17B illustrates the impaction stand of FIG. 17A assembled with the stem component of FIG. 2G.
FIG. 18 is a perspective view of an exemplary taper breaker tool in accordance with an embodiment of the present disclosure.
FIGS. 19A-19M illustrate various individual steps of a method of replacing a proximal portion of the humerus of FIG. 1B.
FIG. 20A illustrates an exemplary surgical navigation system in accordance with the present disclosure.
FIG. 20B is a perspective view of an exemplary bone clamp that may be implemented in the computer assisted surgery system of FIG. 20A and in accordance with an embodiment of the present disclosure.
FIG. 21 is a perspective view of an exemplary humeral prosthesis according to another embodiment of the present disclosure.
FIG. 22 is a perspective view of an exemplary humeral trial prosthesis according to another embodiment of the present disclosure.
FIG. 23A is a perspective view of a modular adapter according to an embodiment of the present disclosure.
FIG. 23B is a perspective view of a humeral head trial according to another embodiment of the present disclosure.
FIG. 23C is an elevational view of a modular humeral head trial assembly including the modular adapter of FIG. 23A and the humeral head trial of FIG. 23B.
FIG. 23D is a perspective view of the modular humeral head trial assembly of FIG. 23C.
FIG. 23E is a cross-sectional view of the modular humeral head trial assembly of FIG. 23C taken along a midline thereof.
FIG. 24 is an elevational view of a humeral trial prosthesis assembly including the humeral trial prosthesis of FIG. 22 and the humeral head trial assembly of FIG. 23C in a first eccentricity configuration.
FIG. 25 is an elevational view of the humeral head trial prosthesis assembly in a second eccentricity configuration.

### DETAILED DESCRIPTION

When referring to specific directions in the following discussion of certain implantable devices, it should be understood that such directions are described with regard to the implantable devices' orientation and position during exemplary application to the human body. Thus, as used herein, the term "proximal" refers to a location closer to an individual's heart, and the term "distal" refers to a location more distant from the individual's heart. The term "inferior" refers to a location closer to the individual's feet, and the term "superior" refers to a location closer to the individual's head. The term "anterior" refers to a location closer to the front of the body or the face, and the term "posterior" refers to a location closer to the back of the body. The term "medial" refers to a location closer the midline of the body, and the term "lateral" refers to a location further from the midline of the body.

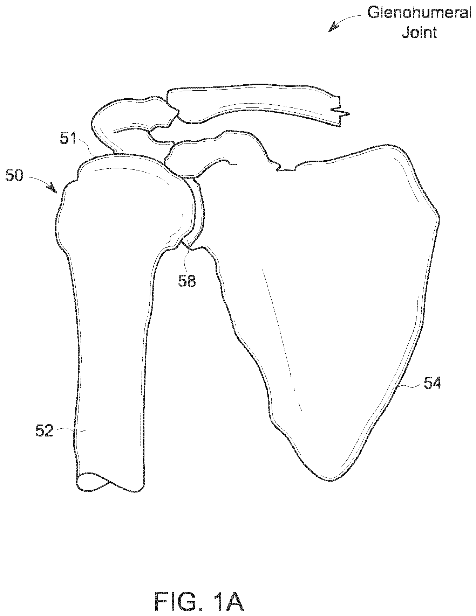

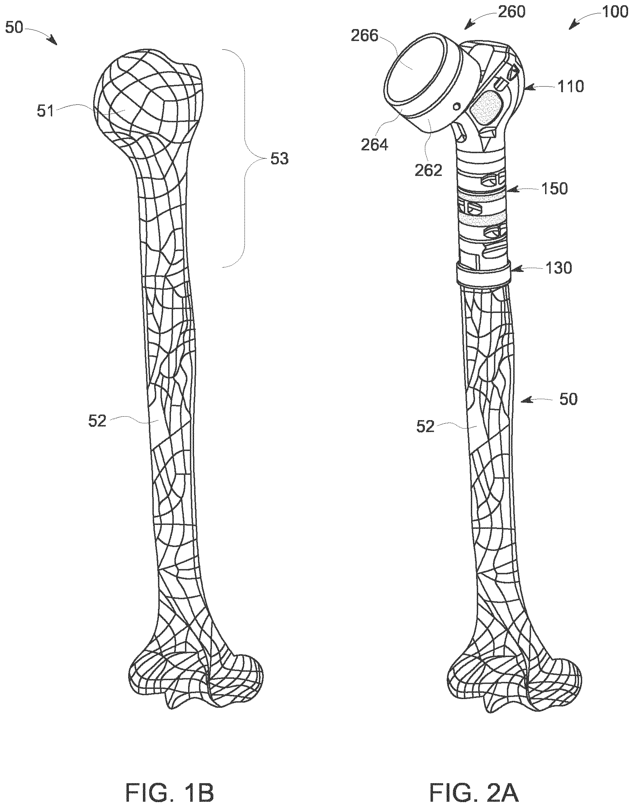

FIGs. 1A and 1B illustrate the anatomy of a typical glenohumeral joint. The joint is formed in part by a head 51 of a humerus 50 and a glenoid 58 of a scapula 54. The head 51 is a convex structure that is generally spherical. The glenoid 58 includes a concave articular surface upon which the head 51 moves. As shown in FIG. 1B, the humerus 50 has a proximal portion 53 which is the portion of the humerus 50 adjacent to the glenoid 58 and forming a part of the shoulder joint. The proximal humerus 53 may sometimes be referred to herein as the superior humerus. When the glenhohumeral joint is arthritic, diseased, or damaged, therapy can include forming an incision over the joint to provide access to the head 51 of the humerus 50 and the glenoid 58 of the scapula 54. Once the head 51 is accessible, the head 51 can be separated from the rest of the humerus 50 as an early part of a method of placing an implant.

In some situations, a significant portion of the proximal or superior humerus 53 may need to be removed or otherwise may be unavailable to support a humeral implant as part of an arthroplasty procedure. For example, in a revision procedure, in which a previously implanted humeral prosthesis must be removed and replaced with a new humeral prosthesis, a significant portion of the proximal humerus 53 may not be available to support the new humeral prosthesis. In another example, major trauma or disease may require a significant portion of the proximal humerus 53 to be removed prior to implanting a humeral prosthesis. In this regard, this significant portion of the proximal humerus 53 may be removed by resecting the humerus 50 through the humeral shaft 52 such that the bone that is removed may include a portion of the humeral shaft 52 and the humeral head 51. As a consequence of removing this bone, the soft tissue attachments of the proximal humerus are also removed. Such soft tissue can include the deltoid muscle and the rotator cuff, which is generally comprised of the supraspinatus, infraspinatus, teres minor, and subscapularis. In order to return functionality to the glenohumeral joint, the resected proximal humerus 53 may be replaced with a humeral prosthesis.

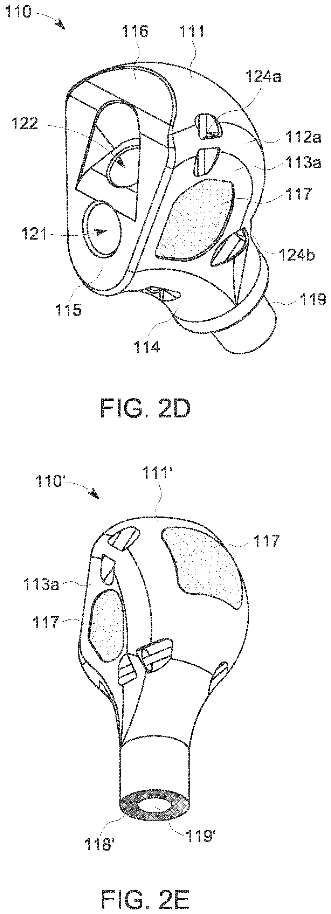

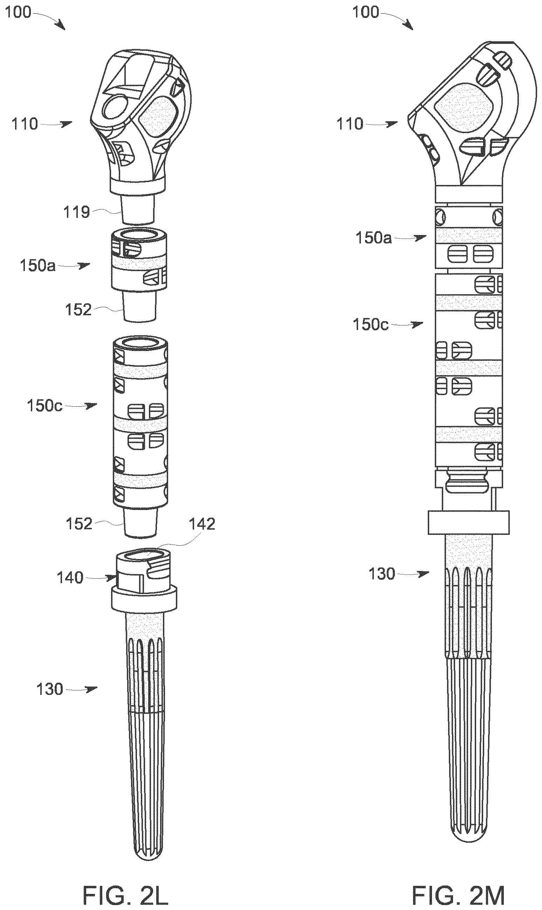

FIG. 2A depicts an exemplary humeral prosthesis 100 that has been implanted into the shaft 52 of the humerus 50 after the proximal portion 53 thereof has been removed. Humeral prosthesis 100 is configured to facilitate the connection of soft tissue thereto and may include a proximal body 110, a stem component 130, and one more implant spacers 150 disposed between proximal body 110 and stem component 130 to account for the length of bone that has been removed. Humeral prosthesis 100 may also include an articular component 260 which may be configured to articulate with a corresponding component implanted into the scapula 54 to replace the glenoid 58. In the embodiment depicted, such articular component 260 is an exemplary reverse articular component that includes a reverse insert tray 262 and a reverse articular insert 264 coupled to reverse insert tray 260. Reverse articular insert 264 includes a concave articular surface 266 configured to articulate with a glenosphere (not shown) which may be implanted onto the scapula 54. Although a reverse articular component 260 is shown connected to proximal body 110, it should be understood that articular component 260 connected to proximal body 110 can alternatively include a humeral head prosthesis (not shown) that may have a convex articular surface configured to articulate with a concave prosthetic glenoid (not shown) implanted onto the scapula 54.

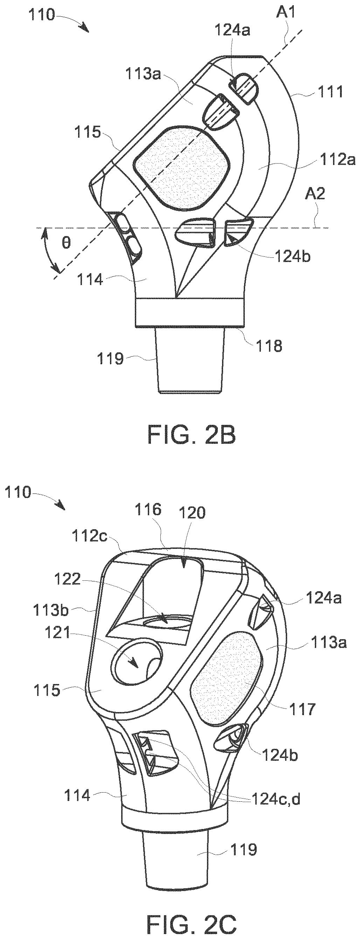

FIGs. 2B-2E depict an exemplary proximal body or prosthesis body 110 that may be implemented in humeral prosthesis 100. Although proximal body 110 generally replaces a proximal end of a humerus 50, proximal body 110 may not be shaped to identically match the bony structure it is intended to replace. Instead, proximal body 110 may be shaped to optimally interact with soft tissue and sutures, wires, and the like which may be used to secure such soft tissue to proximal body 110. In this regard, proximal body 110 may include a plurality of exterior surfaces that together form an exterior geometry of proximal body 110. In the embodiment depicted, proximal body 110 includes a lateral surface 111, a medial surface 114, a first anterior-posterior ("A-P") surface 113a, a second A-P surface 113b, a first proximal surface 115, a second proximal surface 116, and a distal surface 118.

Lateral surface or spherical surface 111 forms a lateral end of proximal body 110. Lateral surface 111 is preferably spherical with a spherical radius of curvature as this geometry facilitates atraumatic soft tissue contact and helps maximize surface area thereby allowing the soft tissue to be spread out over this surface area to ensure functional ranges of motion are achieved. In other words, the deltoid muscle is a triangular shaped muscle that has three heads (i.e., anterior, lateral, and posterior heads) which extend from their origin at the scapula and clavicle over the glenhohumeral joint and to their collective insertion at the deltoid tuberosity which is generally located along the humeral shaft 52. These three heads, in conjunction with other soft tissue, such as the rotator cuff, pectoralis major, and latissimus dorsi, help provide humerus 50 with anterior, lateral, and posterior ranges of motion, respectively. Proximal body 110 may be implanted such that the native deltoid extends over the artificial joint and along lateral surface 111. The spherical geometry of lateral surface 111 facilitates deltoid wrapping so that the three heads of the deltoid spread out over lateral surface 111 and remain in their respective anterior, lateral, and posterior positions encapsulating proximal body 110 so that the forces generated by such muscles are appropriately vectored to provide the desired range of motion. The spherical radius of curvature may vary depending on a size of proximal body 110. For example, the radius of curvature may be 18mm to 24mm.

First and second A-P surfaces 113a-b substantially form the anterior and posterior sides of proximal body 110 and each may intersect lateral surface 111. Proximal body 110 may be utilized in either a right or left shoulder joint such that first and second A-P surfaces 113a-b may each be positioned anterior or posterior within a patient depending on which side of the patient's body proximal body 110 is implanted. First and second A-P surfaces 113a-b are preferably planar surfaces that taper inwardly toward each other from a lateral end of proximal body 110 toward a medial end of proximal body 110. In addition, A-P surfaces 113a-b taper inwardly in a superior to inferior direction. As such, A-P surfaces 113a-b define a dual taper of proximal body 110 which narrows proximal body 110 in a medial direction and an inferior direction.

A-P surfaces 113a-b may each include a porous patch 117 comprised of a porous structure. Such porous structure has a porosity configured to facilitate tissue ongrowth/ingrowth. Porosity is generally a measure of a material's empty space relative to the total space occupied by the material. In contrast, a portion of A-P surfaces 113a-b surrounding each porous patch 117 does not have a porosity or has a porosity of substantially zero. In this regard, while such seemingly non-porous portions of A-P surfaces 113a-b may be considered a solid structure, it is recognized that structures that are seemingly non-porous, at least to the naked eye, may have a porosity on a very small scale. Indeed, structures that are manufactured using an additive manufacturing technique, as may be the case with proximal body, often have an inherent porosity to the material. Thus, as used herein, the terms non-porous and solid mean a porosity so small or so close to zero as to prohibit tissue growth therein. On the other hand, the term porous and the like, as used herein, means having a porosity large enough to facilitate tissue ingrowth. Each porous patch 117, at least due to peaks and valleys that are formed by the porous nature thereof, may have a rough exterior as compared to the nonporous surfaces of proximal portion 110. In this regard, each porous patch 117 may be formed so that it is flush with or recessed relative to the surrounding non-porous portions of A-P surfaces 113a-b. In other words, the peaks of each porous patch 117 may be flush with or recessed relative to the non-porous portions. The planar geometry of first and second A-P surfaces 113a-b in addition to the flush or recessed arrangement of porous patches 117 helps simplify suturing and helps provide clearance for a suture running past porous patches 117 of first and second A-P surfaces 113a-b so that sutures do not rub against a porous patch 117 and become abraded. However, when tissue is compressed against A-P surfaces 113a-b, the tissue comes into contact with porous patches 117 facilitating ingrowth.

Although porous patches 117 are shown and described as being located on A-P surfaces 113a-b, porous patches 117 may be placed in other locations on proximal body 110. For example, as shown in FIG. 2E, an implant proximal body 110' may include a lateral surface 111' with porous patch 117 thereon.

As mentioned, first and second A-P surfaces 113a-b may intersect lateral surface 111. This can form a sharp edge at their respective intersections. To help smooth this edge, the edge may be rounded or chamfered to preclude tissue abrasion. As such, proximal body may include a rounded edge or rounded surface 112a-b extending along respective interfaces between first and second A-P surfaces 113a-b and lateral surface 111.

While first and second A-P surfaces 113a-b may intersect lateral surface 111 at the lateral end of proximal body 110, A-P surfaces 113a-b may also intersect a medial surface 114 at a medial end of proximal body 110. Medial surface 114 may be curved in one or more planes and may be defined by one or more radii of curvature along a superior-inferior length of medial surface 114. It should be noted that a maximum radius of curvature of medial surface 114 is smaller than the radius of curvature defining lateral surface 111.

First and second superior surfaces 115, 116 are each preferably planar surfaces that intersect at an edge 112c which may also be rounded or chamfered. First superior surface 115 is generally oriented at an oblique angle relative to a longitudinal axis of proximal body 110, while second superior surface 116 is generally oriented orthogonal to the longitudinal axis. In the embodiment depicted, first superior surface 115 is intersected by medial surface 114 and first and second A-P surfaces, while second superior surface 116 is intersected by lateral surface 111. However, in other embodiments, first superior surface 115 may also be intersected by lateral surface 111, and in further embodiments, second superior surface 116 may also be intersected by first and second A-P surfaces 113a-b.

Distal surface 118 is disposed at a distal end of proximal body 110 and may be a planar surface, as shown in FIG. 2B. A tapered post 119 may extend from distal surface 118 and may be conically tapered inwardly in a superior to inferior direction. Tapered post 119 may facilitate connection with a correspondingly tapered bore within another component of humeral prosthesis 100, such as an implant spacer 150 and/or implant stem 130, for example. However, other connection mechanisms are contemplated, such as a threaded or snap-fit mechanism, for example. In another example shown in FIG. 2E, proximal body 110' may include a ridged distal surface 118' and a tapered opening 119' to receive a tapered post therein.

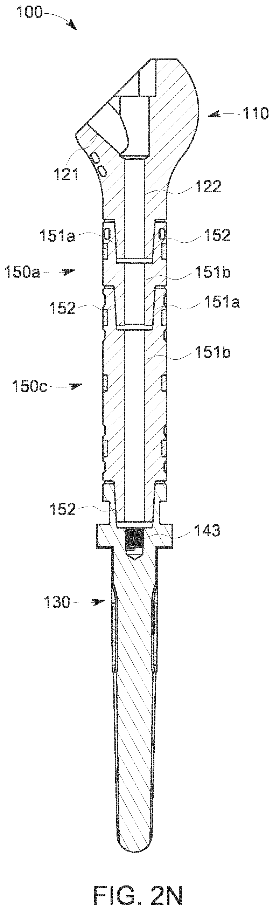

Proximal body 110 may also include first and second bores 121, 122. First bore 121 may extend through first superior surface 115 perpendicular thereto and may intersect second bore 122 at an oblique angle, as shown in FIG. 2N. First bore 121 may be a smooth bore and may be tapered so as to form a taper-lock with a corresponding tapered post of a joint component, such as joint component 260, for example.

Second bore 122 may be a smooth bore and may extend entirely through proximal body 110 in a superior inferior direction, as best shown in the cross-sectional view of FIG. 2N. In this regard, second bore 122 may extend through first and/or second proximal surfaces 115, 116 at a superior end of proximal body 110 and through tapered post 119 at an inferior end of thereof. To facilitate access to second bore 122, a cutout or recess 120 surrounding second bore 122 may extend through first and second proximal surfaces 115, 116, as best shown in FIG. 2C.

In addition to porous patches 117, which may facilitate tissue ongrowth/ingrowth into proximal body 110, proximal body 110 may also include a plurality of suture holes or suture passages 124 which may facilitate connection of soft tissue to proximal body 110 via sutures, wires, and the like. In particular, both the anterior and posterior sides of proximal body 110 may include a first suture hole 124a and a second suture hole 124b. As shown in FIG. 2B, first suture hole 124a may extend through lateral surface 111 and first A-P surface 113a traversing the interface 112a thereof. Such suture hole 124a may define an axis A1 that extends both laterally-medially and superiorly-inferiorly. Second suture hole 124b may be located more distal to first suture hole 124a and may also extend through lateral surface 111 and first A-P surface 113a traversing their interface 112a. An axis A2 defined by second suture hole 124b may extend substantially in a lateral-medial direction. However, in other embodiments axis A2 may extend in the superior-inferior ("S-I") direction as well as the lateral-medial ("L-M") direction.

In some embodiments, axis A1 and axis A2 may lie in the same plane and intersect at an angle Θ, which may be an acute angle, such that axis A1 and axis A2 form an "X" shape configuration. For example, angle Θ can be 20 to 60 degrees. However, in other embodiments, axis A1 and axis A2 may be offset from each other in the A-P direction such that they are not coplanar. In such an arrangement, axis A1 and axis A2, although not intersecting, may nonetheless be projected into a single plane, such as the plane defined by FIG. 2B, so as to form the same X-shape configuration just mentioned. It should be understood that the same arrangement of first and second suture openings 124a-b is also included on the other A-P side of proximal body 110 and with respect to second A-P surface 113b as that shown in FIG. 2B.

Proximal body 110 may also include second and third suture holes 124c-d extending through medial surface 114 substantially in the A-P direction, as best shown in FIGs. 2B and 2C. Although, proximal body 110 is shown and described as having six suture holes (i.e., 2-pairs of first and second suture holes 124a-b and third and fourth suture holes 124c-d), other embodiments of proximal body 110 may include more or fewer suture holes 124.

The X-shape of the intersecting axes A1, A2 of first and second suture holes 124a-b either alone or in combination with third and fourth suture holes 124c-d facilitates compression of soft tissue against porous patches 117. For example, a subscapularis may be sutured to an anterior side of proximal body 110, while an infraspinatus may be sutured to a posterior side of proximal body 110. This may be achieved by placing such muscles against respective porous patches 117 of first and second A-P surfaces 113a-b and threading one or more sutures through first and second openings 124a-b at the anterior and posterior sides of proximal body 110, through one or both of third and fourth suture openings 124c-d at the medial end of proximal body 110, and over the muscles, for example. The one or more sutures in this regard may extend along axes A1 and A2 and traverse about proximal body 110 such that the subscapularis and infraspinatus are compressed by sutures against the entire patch area of porous patches 117 at the anterior and posterior sides of proximal body 110, respectively. In a further example, a supraspinatus may also be secured to by sutures extending through the suture openings 124a-b and compressed against lateral surface 111. The X-shape defined by openings 124a-b forms intersecting force vectors, which extend along axes A1 and A2, that help compress the supraspinatus muscle against lateral surface 111 and against patch 117 on lateral surface 111, when included, to facilitate ongrowth/ingrowth of the supraspinatus.

Additionally, axes A1 and/or A2 of the X-shape extend along planar A-P surfaces 113a-b which not only provides the benefits mentioned above, such as abrasion avoidance, but it also allows a needle to pass through suture holes 124a-b without a step or other material obstruction in its path. In other words, a suture needle passing through either suture hole 124a-b and soft tissue has a clear path at least due to the planarity of A-P surfaces 113a-b.

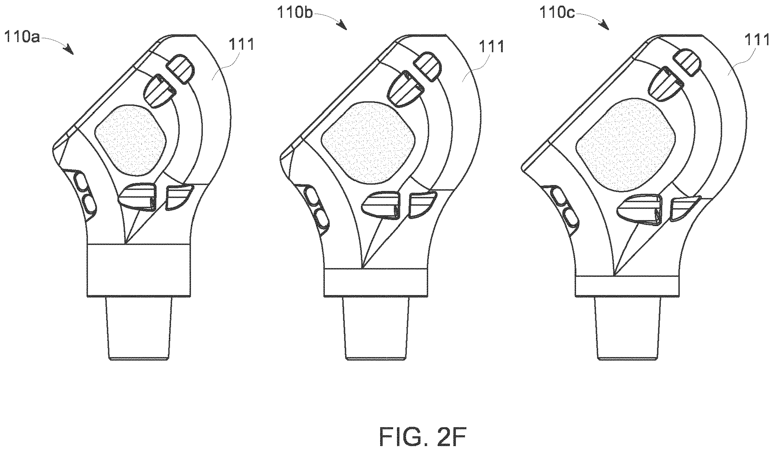

A kit may include multiple proximal bodies 110 each differing in size. For example, a kit may be provided which may include two or more proximal bodies 110 of different sizes. In another example, as shown in FIG. 2F, a kit may be provided with a first proximal body 110a, a second proximal body 110b, and a third proximal body 110c where such proximal bodies 110a-c may be characterized as small, medium, and large. The size differences between these proximal bodies 110a-c can include differences in A-P and medial-lateral ("M-L") dimensions. In addition, each of the differently sized proximal body 110a-c may have a lateral surface 111 with a different radius of curvature than the others. For example, lateral surface 111 of first, second, and third proximal bodies 110a-c respectively may have a radius of curvature of 18mm, 20mm, and 22mm. However, in other embodiments, one or more of proximal bodies 110a-c may have the same radius of curvature. For example, lateral surface 111 of first and second proximal bodies 110a-b may have a radius of curvature of 22mm.

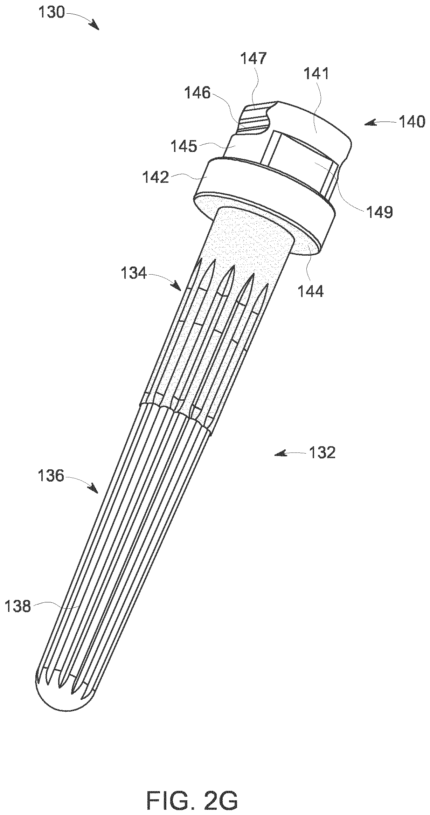

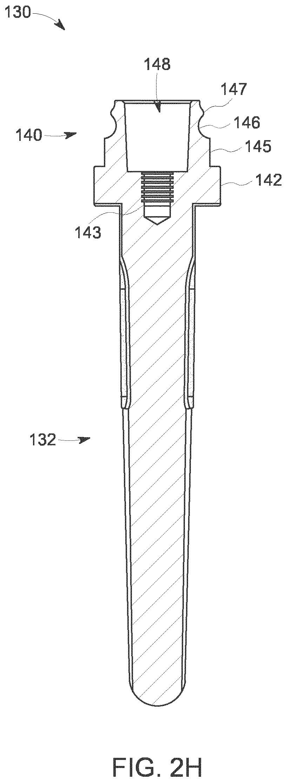

FIGs. 2G and 2H depict an exemplary implant stem component 130 according to an embodiment of the present disclosure. Stem component 130, in the embodiment depicted, is a monoblock stem component that includes a stem 132 and an adapter 140 integrally connected to stem 132 so as to form a monolithic structure. However, in other embodiments, stem 132 and adapter 140 may be separate components modularly connected, examples of which are described in U.S. Application No. 18/189,261 ("the '216 Application"), which is incorporated by reference herein in its entirety.

Stem 132 is configured to be inserted in an intramedullary canal of the remaining humeral shaft 52. Stem 132 may include features that are configured to mate with humeral shaft 52 to prohibit rotation relative thereto. For example, stem 132 may be configured for a press-fit connection and may include a plurality of flutes or splines 138, as shown in FIG. 2G, which may enhance primary engagement and rotational resistance with the bone. Additionally, stem 130 may include a porous structure to facilitate ingrowth and secondary engagement with bone. For example, stem 132 may include a proximal portion 134 and a distal portion 136. Proximal portion 134 may have a porous structure comprising an exterior thereof, while distal portion 136 may be substantially solid. For example, the porous structure of proximal portion 134, and all other porous structures mentioned herein, may comprise an on-growth structure, such as a titanium plasma spray, for example, or an ingrowth structure, such as a porous structure formed from an additive manufacturing process, such as 3D printing, and the like. Flutes 138 may extend both proximal and/or distal portions 134, 136. In other embodiments, stem 132 may be configured to be cemented into humeral shaft 52 and may not include a porous structure. However, ridges, flutes, splines, or the like may be provided to promote interdigitation of bone cement to reduce or eliminate rotation of stem 132 within humeral shaft 52.

Adapter 140 may be a generally cylindrical member and may include a radially protruding distal collar 142 configured to abut the proximal end of the humeral shaft 52. Distal collar 142 may include a distal surface 144 having a porous structure to promote bone ingrowth from the resected proximal end of humeral shaft 52. Adapter 140 may also include a base portion or adapter body 145 extending proximally from distal collar 142. As shown in FIG. 2H, a tapered bore 148 may extend into adapter body 145 through a proximal end thereof. Such tapered bore 148 may be configured to mate with a tapered post of another component of humeral prosthesis 100, such as tapered post 119 of proximal body 110, for example. A threaded opening 143 may extend through a distal end of tapered bore 148. Such threaded opening 143 may be configured to engage a screw of such other humeral prosthesis component to help secure it thereto, and/or may be configured to connect a removal tool (e.g., slap hammer) for explantation, for example.

Connection features may be formed on an exterior of adapter body 145. For example, adapter 140 may be configured to connect to an inserter/extractor handle 700, which is described in more detail below. In this regard, adapter 140 may include opposing flats 149 each extending perpendicular to a longitudinal axis of stem component 130 and may be bounded at a distal end by distal collar 142 and a proximal end by a proximal collar or shoulders 141, which may have a cross-sectional dimension smaller than distal collar 142.

Other connection features may include features configured to quickly connect and disconnect from other components, such as trial proximal bodies 310, trial spacers 350, and a height gauge 800, for example, each of which are described further below. In this regard, adapter 140 may include two divots 146, with each divot 146 being bound at its proximal end by a shoulder or protrusion 147 and at its distal end by the diameter of adapter body 145. In the illustrated embodiment, the divots 146 are rotationally offset from flats 149 by about 90 degrees, but in other embodiments, other relative orientations may be suitable.

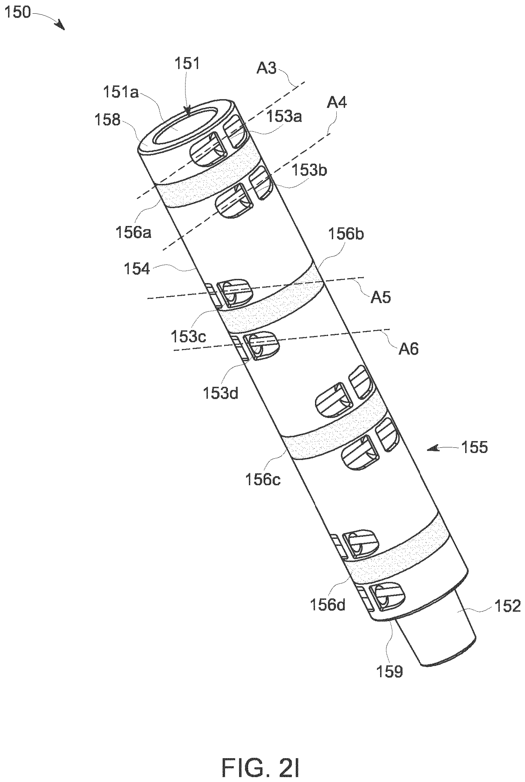

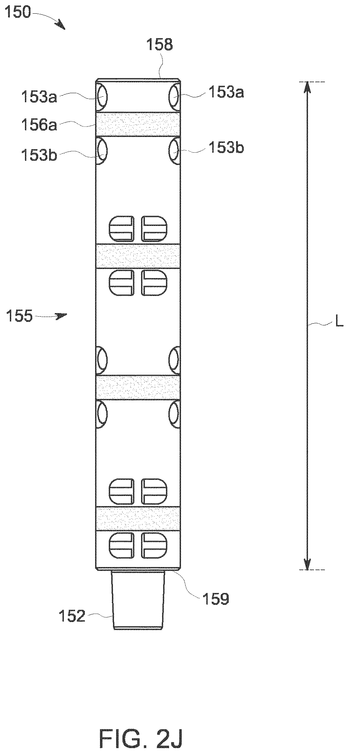

In order to build up the humeral prosthesis 100, one or more spacers 150 may be connected to proximal body 110 and implant stem component 130 to achieve the desired height. FIGs. 2I and 2J depict an exemplary implant spacer 150 that may be implemented in humeral prosthesis 100. Implant spacer 150 may include a spacer body 155 and a tapered post 152 extending distally from body 155.

Spacer body 155 may be substantially cylindrical. However, other shapes are contemplated, such as a rectangular shape, for example. Spacer body 155 may extend from a proximal surface 158 to a distal surface 159, and a length L of spacer may be measured from proximal surface 158 to distal surface 159. Length L of spacer 150 at least partially determines a height of humeral prosthesis 100, as described further below.

Spacer body 155 may be substantially formed of a solid metallic material. However, spacer body 155 may include a plurality of porous rings 156 positioned at regular intervals along its height. For example, as shown, spacer 150 may include four porous rings 156, namely a first porous ring 156a, a second porous ring 156b, a third porous ring 156c, and a fourth porous ring 156d. However, spacer 150 may have more or fewer porous rings 156. For example, shorter implant spacers 150 may have fewer rings 156, such as one to three rings 156, and longer spacers 150 may have more rings 156, such as five to eight rings 156. Additionally, although these porous structures are shown and described as rings that circumferentially extend about a longitudinal axis of spacer body 155, other embodiments of spacer 150 may include porous patches that do not extend entirely about spacer body 155. As mentioned above, removal of proximal humerus 153 may also remove tissue attachments. Porous rings 156 facilitate tissue reattachment to humeral prosthesis 100 via tissue ingrowth.

Spacer body 155 may also include a plurality of suture openings 153 which may further facilitate tissue attachment via sutures, wires, and the like. In the embodiment depicted, suture openings 153 may be arranged relative to porous rings 156 so that suture openings 153 may be used to compress tissue against porous rings to facilitate tissue ingrowth. In this regard, a first suture opening 153a may be positioned proximal and adjacent to a first porous ring 156a, and a second suture opening 156b may be positioned distal and adjacent to first porous ring 156a. First suture opening 156a may define an axis A3, and second suture opening 153b may define a second axis A4. Axes A3 and A4 may extend perpendicular to a longitudinal axis of spacer body 155 and may extend parallel to each other. However, in some embodiments, axes A3 and A4 may be angled relative to each other such that they intersect. In this arrangement of first and second suture openings 153a-b and first porous ring 156a, tissue may be sutured at proximal and distal sides of suture ring 156a such that the tissue is compressed onto porous ring 156a to facilitate tissue ingrowth.

First and second suture openings 153a-b are positioned at one side of spacer body 155, such as an anterior side, for example. Another pair of first and second openings 153a-b may be positioned at an opposite side of spacer body, such as at a posterior side thereof, as illustrated in FIG. 2J. In such an arrangement, axes of the second pair of first and second suture openings 153a-b may be parallel to respective axes A3 and A4 of the first pair of first and second suture openings 153a-b. However, in some embodiments, the second pair of suture openings 153a-b may not be rotationally offset 180 degrees relative to the first pair of openings 153a-b such that their respective axes intersect.

The arrangement of first and second suture openings 153a-b proximal and distal to first porous ring 153a may be applied to the other suture rings, such as rings 156b-d, disposed along spacer body 155. However, as shown in FIGs. 2I and 2J, suture openings 153 associated with each of the rings 156a-d of spacer body 155 may be rotationally staggered relative to suture openings 153 of other rings proximal and distal thereto. For example, third and fourth suture openings 153c-d associated with a second suture ring 156b are rotated 90 degrees relative to first and second suture openings 153a-b such that respective axes A5 and A6 of third and fourth suture openings 153c-d are oriented perpendicular to axes A3 and A4 of first and second suture openings 153a-b. Such staggered arrangement may provide maximum flexibility to the surgeon for soft tissue attachment. However, it should be understood that in other embodiments, third and fourth suture openings 153c-d may not be rotationally staggered relative to first and second suture openings 153a-b such that axes A5 and A6 may be parallel to axes A3 and A4.

Additionally, a bore 151 may extend axially entirely through spacer 150 including tapered post 152. Such bore 151 may have a first portion 151a and a second portion 151b, as shown in FIG. 2N which is a cross-sectional view humeral prosthesis 100 with other spacers (i.e., spacers 150a and 150c) with the same bore features. First portion 151a of bore 151 may be located at a proximal end of spacer 150 and may be conically tapered to accommodate a tapered post 152 of another spacer 150 or tapered post 119 of proximal body 110, for example. Second portion 151b may be substantially cylindrical and may extend from first portion 151a toward a distal end of spacer 150 extending through post 152. This allows a tool to traverse spacer 150.

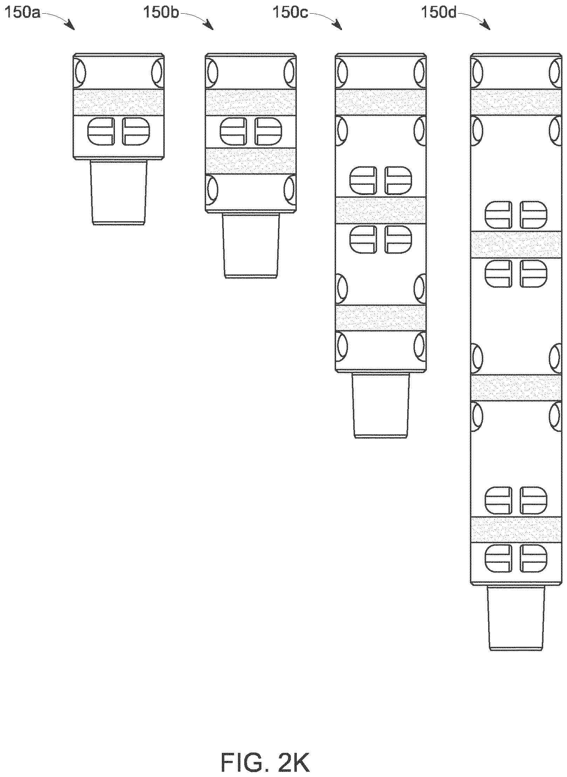

As with other components described herein, although a single implant spacer 150 is shown in FIGs. 2I and 2J, it should be understood that a plurality of implant spacers 150 may be provided in a kit, of the same or differing heights. For example, FIG. 2K depicts a plurality of implant spacers 150 of differing height that may be provided in a kit, such as a first implant spacer 150a, a second implant spacer 150b, a third implant spacer 150c, and a fourth implant spacer 150d. Each of these spacers 150a-d may have the same features as described above with respect to implant spacer 150 with the exception that the spacer bodies thereof differ in height. For example, where the four implant spacers 150a-d are provided in a kit, such implant spacers 150-ad may be provided with heights of 20mm, 30mm, 60mm, and 100mm, respectively, to accommodate a variety of clinical situations in the operating theater. Additionally, at least due to the height differences between implant spacers 150a-d, shorter spacers may have fewer suture openings 153 and porous rings 156 than longer spacers, as illustrated in FIG. 2K.

FIGs. 2L to 2N illustrate the assembly of a humeral prosthesis 100. In the particular embodiment depicted, humeral prosthesis 100 includes proximal body 110, stem component 130, and first and third spacer 150a, 150c of the above-mentioned kit. Thus, humeral prosthesis 100 of FIGs. 2L to 2N demonstrates that more than one spacer 150 may be utilized to provide the desired height to humeral prosthesis 100. Humeral prosthesis 100, and the height thereof, may be assembled by stacking each component onto a next component. As shown, third spacer 150c may be connected to adapter 140 of stem component 130 by inserting tapered post 142 into tapered bore thereof. Similarly, first implant spacer 150a may be connected to third implant spacer 150c by inserting tapered post 152 of first implant spacer 150a into first portion 151a of bore 151 of third implant spacer 150c. Finally, proximal body 110 may be connected to first implant spacer 150a by inserting tapered post 152 of proximal body 110 into first portion 151a of bore 151 of first implant spacer 150a. Each of these connections form a series of Morse taper locks that secure each component to the next. Also, as shown in the cross-sectional view of FIG. 2N, the bores 122 and 151 of proximal body 110 and implant spacers 151a, 150c, respectively, align so as to form a bore that extends the entire height of humeral prosthesis 100 to stem component 130. This may allow a driver to extend through the stack distally to stem component 130 so as to drive a screw into threaded opening 143 of stem component 130 to provide additional securement between stem component 130 and third implant spacer 150c, for example.

When assembled, the through-bores 151b of each spacer 150a, 150c and second bore 122 of proximal body 110 align so as to collectively form a through-bore which extends entirely through proximal body 110 and spacers 150a, 150c. This may allow a driver with a screw, for example, to be inserted through proximal body 110 and spacers 150a, 150c down to stem component 130. The screw may engage threaded opening 143 in adapter 140 of stem component 130 and the distal most spacer 150c to further secure the distal most spacer 150c to implant stem component 130.

Once humeral prosthesis 100 is assembled and implanted, it may be desirable to trial the particular constructed configuration to ensure the desired articulation is achieved and prior to finalizing the construction of prosthesis 100 by attaching the articular component, such as component 260, thereto.

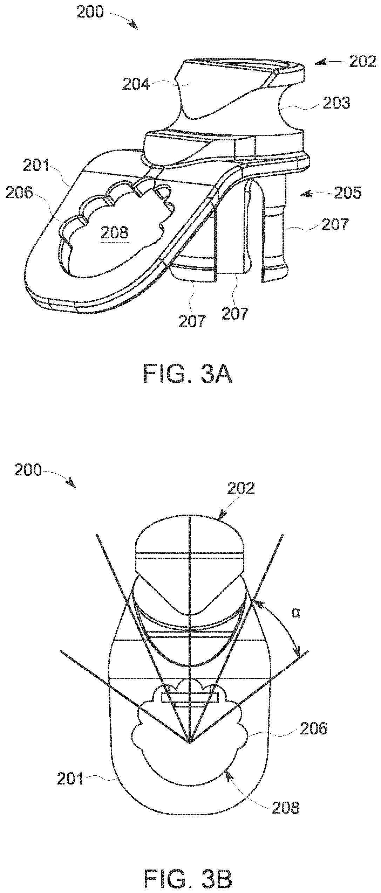

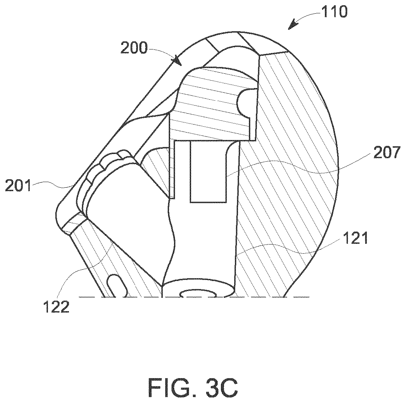

FIGs. 3A-3B depict a proximal body trialing adapter 200 that may be implemented with proximal body 110 to facilitate trialing of an articular component in conjunction with humeral prosthesis 100. Proximal body trialing adapter 200 may include a post 202, a flange 201 extending radially outwardly from post 202, and a connection portion 205 extending distally from post 202. Post 202 may include an inclined surface 204 and a circumferentially extending groove 203. Connection portion 205 may include a plurality of moveable legs or flexures 207. For example, three legs 207 may be provided which may be arrayed in an evenly spaced arrangement about a longitudinal axis of adapter 200. However, in other example, more or fewer legs 207 may be provided. As shown in FIG. 3C, connection portion 205 and post 202 may be inserted into second bore 122 of proximal body 110. When inserted, legs 207 may be pushed radially inwardly by the surface that defines second bore 122 which may have a smaller diameter than a maximum diameter defined by legs 207 in a relaxed, unflexed state. In this regard, legs 207 may press against proximal body 110 from within bore 122 to secure adapter 200 therein. In this arrangement, inclined surface 204 of post 202 may be flush with or recessed relative to the inclined first proximal surface 115 of proximal body 1110 so as to provide clearance for a trial articular component. Once trialing is completed, proximal body trialing adapter 200 may be removed by gripping post 202 and pulling trialing adapter 200 out of second bore 122. This may be assisted by groove 203 which forms a finger-pull feature.

Flange or tab 201, while extending radially from post 202, also extends at an inclined angle in a distal direction away from post 202. Such inclined distal extension allows flange 201 to extend over first bore 121 when adapter 200 is inserted within second bore 122, as best shown in FIG. 3C. Flange 201 may include an opening 208 that extends entirely through a thickness thereof. Opening 208 may be a scalloped opening or splined opening. In this regard, a plurality of scallops or splines 206 may be arrayed about opening at a predetermined increment which may be defined by an angle α, as shown in FIG. 3B. In other words, each scallop 206 may be rotationally offset from an adjacent scallop by angle α measured from a center point of opening 208. Increment angle α may be 30 degrees, for example, but may also be 5 to 45 degrees. As discussed below, such scallops 206 allow an articular component received within opening 208 to be rotated at regular intervals to produce a desired eccentricity relative to proximal body 110, and in particular, first bore 121. In the particular embodiment depicted, seven scallops 206 are provided. However, more or fewer scallops 208 are contemplated, such as 3 to 12 scallops, for example.

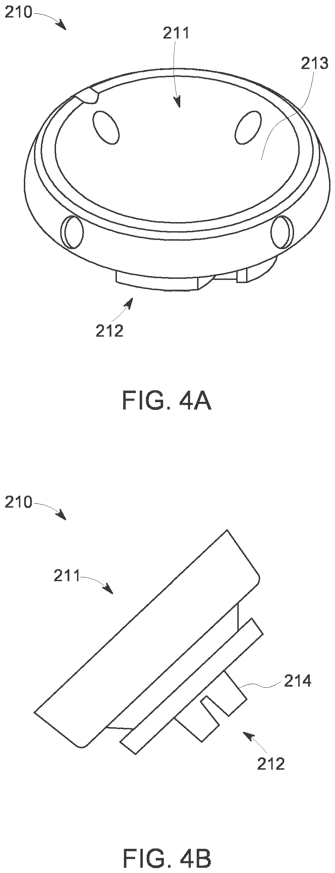

FIGs. 4A-5G depict an exemplary first trial articular component which includes a reverse trial insert 210 and a trial tray 220. Thus, the trial articular component is a reverse trial articular component. FIGs. 4A and 4B depict an exemplary reverse trial insert 210 which may include an articular side 211 and a connection side 212. Articular side 211 may include a concave articular surface 213 that may be configured to articulate with a trial glenosphere (not shown). Connection side 212 may be configured to connect to trial tray 220. In this regard, connection side 212 may include a pair of legs or flexures 214 that may be configured to fit within an opening or recess within trial tray 220, such as recess 227, which is described further below. However, other connection mechanisms are contemplated, such as snap-fit or twist-lock mechanism, for example.

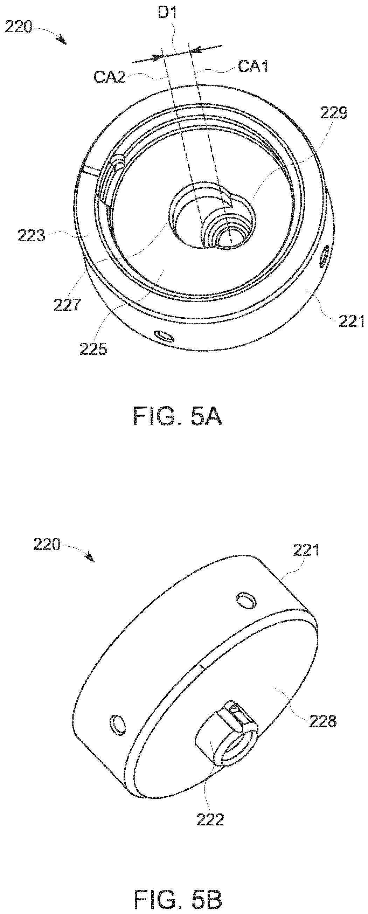

FIGs. 5A-5G depict an exemplary trial tray 220. Trial tray 220 may include a tray body or trial body 221 and a boss 222 extending distally from tray body 221. Tray body 221 may include a circumferentially extending rim 223 and an inner surface 225 bounded by rim 223 such that rim 223 and inner surface 225 together may define a cavity for receipt of at least a portion of connection side 212 of reverse trial insert 210. A central opening or recess 227 may at least partially extend into tray body 221 and through inner surface 225 such that a central axis CA2 of opening 227 is coaxial with a central axis of trial tray body. A through bore 229 extends through tray body 221 and overlaps with opening 227 such that a central axis CA1 of through-bore 229 is offset from central axis CA2 of opening 227 by a distance D1, as shown in FIG. 5A. Tray body 221 may also include a planar distal surface 228 disposed opposite inner surface 225.

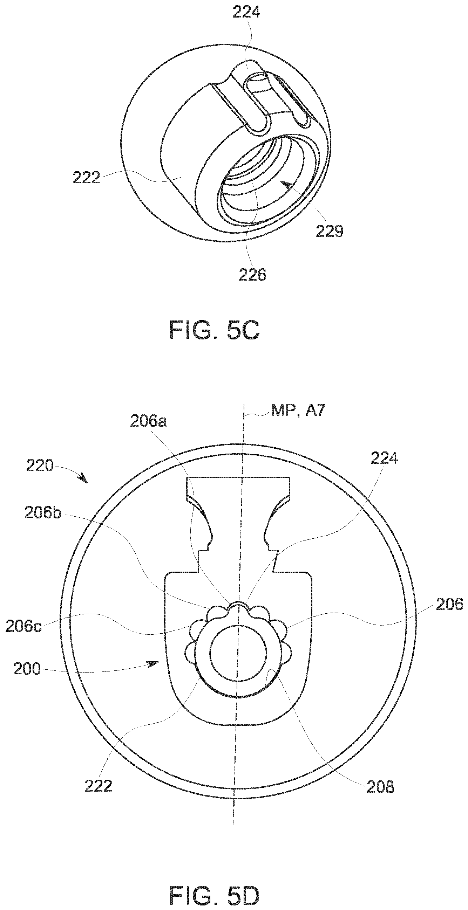

Boss 222 extends distally from distal surface 228. Through-bore 229 extends through boss 222 such that a central axis of boss 222 is also a central axis of through-bore 229. In other words, central axis CA1 is the central axis for through-bore 229 and boss 222. Through-bore 2229 may have threading 226 along at least a portion of its length, as best shown in FIG. 5C. Boss 222 may also include a tooth or protrusion 224 extending radially outwardly therefrom. Such tooth 224 may be configured to be received in and engage any one of scallops 206 of proximal body trialing adapter 200 when boss 222 is received within scalloped opening 208 thereof, as best shown in FIG. 5D. Tooth 224 may be a stationary tooth such that boss 222 may be required to be removed from scalloped opening 208 and out of engagement with one scallop 206 in order to engage another scallop 206. However, in other embodiments, tooth 224 may be moveable radially inwardly such that applying threshold amount of torque to tray 220 while boss 222 is disposed within scalloped opening 208 would cause tooth 224 to move inwardly allowing tray 220 to rotate to another clocking position within opening 208 at which location tooth 224 may snap back into position and into engagement with another scallop 206. In either embodiment, tooth-and-scallop engagement generally prohibits rotation of trial tray 220 relative to adapter 200 such that boss 222 is rotationally secured within scalloped opening 208.

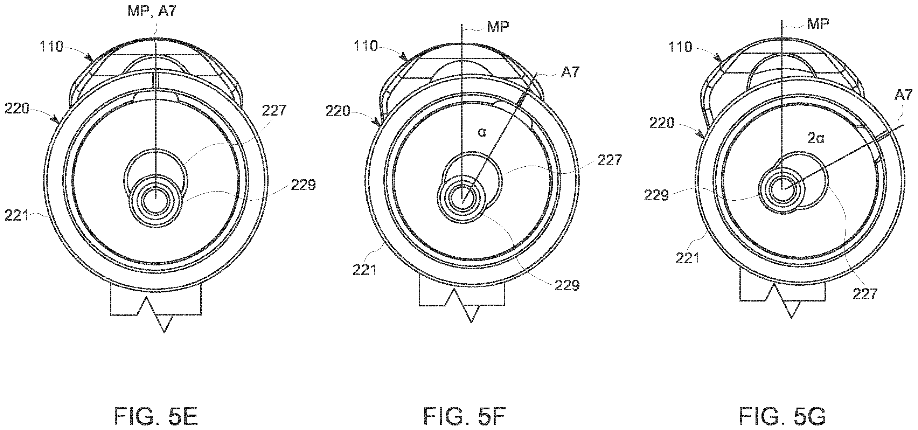

As illustrated by FIGs. 5E-5G, trial tray 220 may have a plurality of eccentricity configurations relative to implant proximal body 110. Such eccentricity configurations may, at least partially, be determined by which scallop 206 tooth 224 engages. For example, FIGs. 5D and 5E illustrate a first eccentricity configuration in which tooth 224 engages a first scallop 206a. In this first eccentricity configuration, an axis A7 extending through tooth 224 and the first scallop 206a is coplanar with a midline plane MP which bisects proximal body 110 and extends in the S-I direction and substantially in the M-L direction. Thus, in this configuration the central axes CA1, CA2 of through-bore 229 and central opening 227 are coplanar with the midline plane MP such that trial tray 220 exhibits no eccentricity relative to proximal body 110.

This is in contrast to a second eccentricity configuration, as depicted in FIG. 5F, in which trial tray 220 is rotated from the previously mentioned first configuration by the angle α such that tooth 224 engages a second scallop 206b adjacent to first scallop 206a. Thus, in this second eccentricity configuration, axis A7, which now extends through tooth 224 and second scallop 206b, intersects the midline plant MP by the angle α. Additionally, although central axis CA1 of through-bore 229 is coplanar with the midline plane MP, the central axis CA2 of central opening 227 is not. As such, central axis CA2 of central bore is offset from midline plane in the A-P direction resulting in an eccentric relationship between trial tray 220 and proximal body 110. This translates into an eccentric positioning of articular surface 221 of trial insert 210 when coupled to trial tray 220.

FIG. 5G depicts a third eccentricity position which demonstrates an increase in eccentricity relative to the previous first and second eccentricity configurations. As shown, tooth 224 is rotated within scalloped opening 208 to engage a third scallop 206c adjacent to second scallop 206c. In this regard, axis A7 intersecting this third scallop 206c and tooth 224 intersects the midline plane by an angle 2α. In this position, central axis CA2 of central opening 227 is offset even further from midline plane MP in the A-P direction than in the second eccentricity configuration. It should be noted that eccentricity may be the greatest when axis A7 is perpendicular to the midline plane MP.

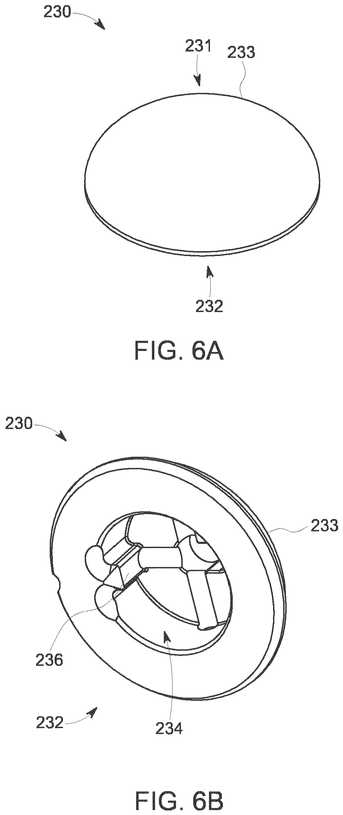

FIG. 6 depicts an exemplary humeral head trial 230 that may be implemented with humeral prosthesis 100 and in accordance with an embodiment of the present disclosure. Humeral head trial 230 may include an articular side 231 and a connection side 232. Articular side 231 may include a convex articular surface 233 that may be partially spherical and configured to articulate with a corresponding concave glenoid prosthesis or trial component. Connection side 232 may be configured to separately connect to a humeral head coupler trial, such as humeral head coupler trials 250 described in more detail below. In this regard, connection side 233 may include a socket 234 and a tab or rib 236 extending radially inwardly into socket 234, as shown in FIG. 6B.

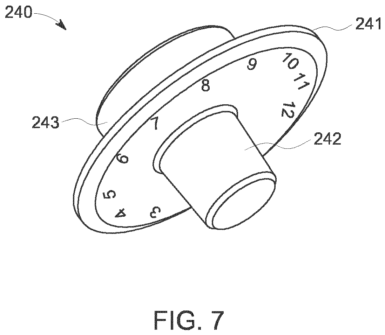

Humeral head trial 230 is also representative of a humeral head prosthesis which may be configured to couple with a humeral head coupler, such as humeral head coupler 240 shown in FIG. 7. Humeral head coupler 240 may include a connector 243, a plate or flange 241, and a tapered post 242. Tapered post 242 may be configured to be received within second bore 122 of proximal body 110. Additionally, connector 240 may be configured to connect to connection side 232 of humeral head 230. For example, connector 240 may be a circular boss configured to be received within socket 234 of humeral head 230.

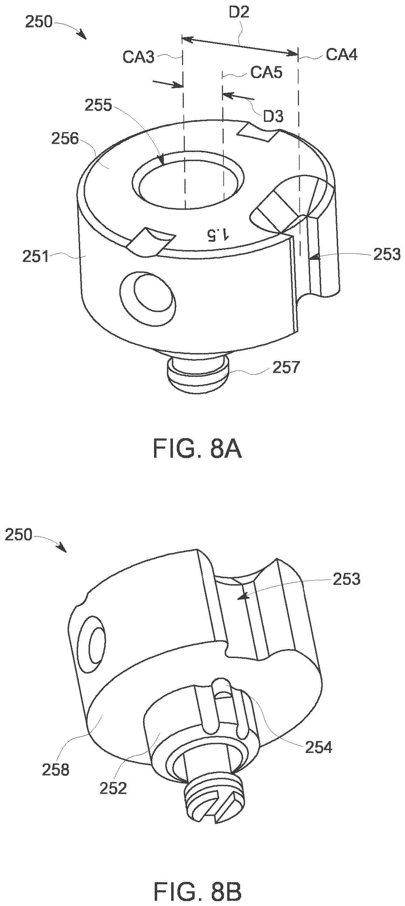

FIGs. 8A and 8B depict an exemplary humeral head coupler trial 250 according to an embodiment of the present disclosure. Coupler trial 250 may include a connection body 251 and a boss 252 extending from connection body 251. Connection body or trial body 251 may be configured to engage connection side 232 of humeral head trial 230 to secure humeral head trail 230 thereto. For example, socket 234 of humeral head trial 230 at connection side 232 thereof may be correspondingly sized and shaped to receive trial body 251 therein. Trial body 251 may include a through-bore 255 and an axial slot 253. Axial slot 253 may extend into a side of trial body 251 and from a first surface or superior surface 256 to a second surface or inferior surface 258. Axial slot 253 may be configured to receive a feature, such as tab 236, within connection side 232 of humeral head trial 230 to prevent relative rotation therewith. Through-bore 255 may define a central axis CA3 that is offset from a central axis CA4 of axial slot 253 by a distance D2 which may correspond to a degree offset applied to humeral head trial 230 when coupled to coupler trial 250. For example, coupler trial 250 may have an offset of 3.5mm. Other offsets are possible though, such as a 1.5mm offset or a centered offset (i.e., no offset), for example. Thus, a kit may include a plurality of coupler trials 250 each with a different offset. Additionally, trial body defines a central axis CA5 which extends through a center point of trial body. Central axis CA5 is offset from through-bore central axis CA3 by a distance D3.

Boss 252 may be similar to boss 222 of trial tray 220. In this regard, boss 252 may have a tooth or protrusion 254 which may be configured to engage any of scallops 206 in proximal body trialing adapter 200 so as to position humeral head coupler trial 250 and humeral head trial 230 coupled thereto in any one of a plurality of eccentricity configurations, such as those described above, for example. Also, similar to trial tray 220, through-bore 255 extends through boss 252 such that central axis CA3 of through-bore 255 is coaxial with boss 252. As illustrated in FIGs. 8A and 8B, coupler trial 250 may be secured to implant proximal body 110, or another device, such as trial proximal body 230 described below, via a screw 257 positioned within through-bore 255.

Although humeral prosthesis 100, as described above, may be adapted to provide a trialing functionality, it is typically desirable to first implant "trial" components that represent the functionality of humeral prosthesis 100. In this regard, a trial humeral prosthesis may be constructed and implanted within humeral shaft. With the trial prosthesis in place, range of motion, joint positioning, and other parameters that may be predictive of success may be tested or otherwise determined. When the surgeon determines that a particular trial implant is indicative of desirable surgical outcomes, the actual humeral prosthesis 100 may be implanted in a configuration that strives to match the successful trial configuration, so that the final permanent implant has the desirable parameters tested or identified with the corresponding trial configuration.

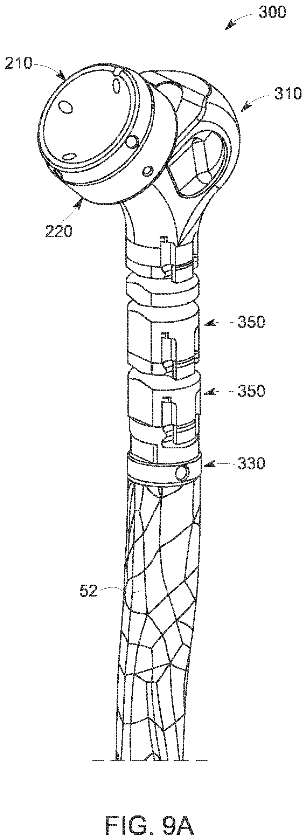

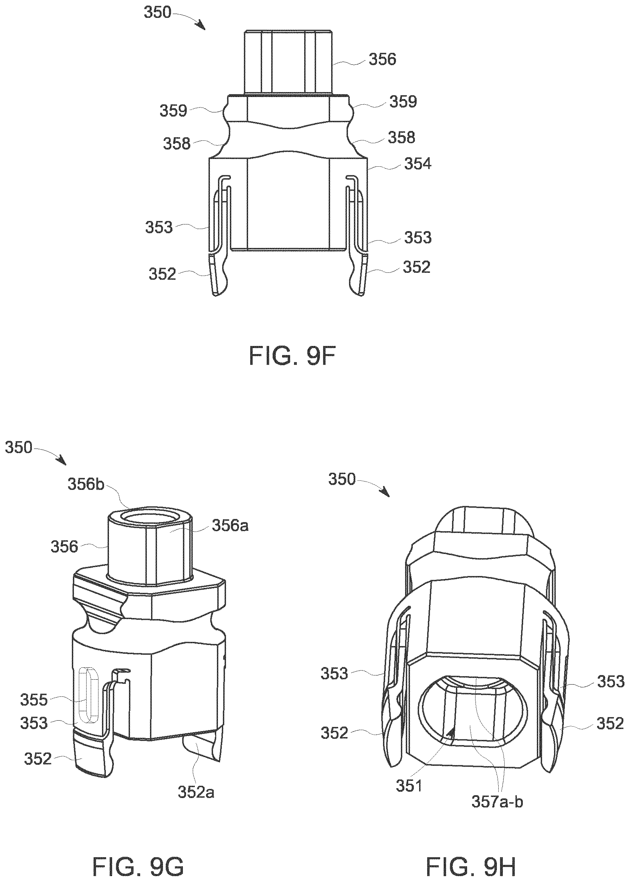

FIG. 9A depicts an exemplary humeral trial prosthesis 300 according to an embodiment of the present disclosure and as implanted within humeral shaft 52. Humeral trial prosthesis 300 may include a trial proximal body 310, an expandable trial stem component 330, and one or more trial spacers 350 disposed between trial proximal body 310 and expandable stem component 330 to account for the length of bone that has been removed.

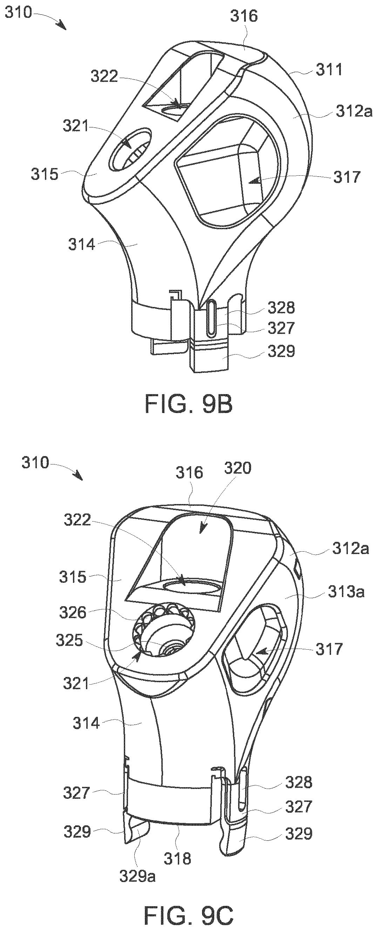

FIGS. 9B-9D depict an exemplary trial proximal body or trial prosthesis body 310 according to an embodiment of the present disclosure. Trial proximal body 310 corresponds with implant proximal body 110. As such, trial proximal body 310 has similar features to that of implant proximal body 110. For ease of review, like elements of trial proximal body 310 are accorded like reference numerals to that of implant proximal body 110 but within the 300-series of numbers. For instance, trial proximal body 310 may have a similar exterior geometry to that of implant proximal body 110 such that it has a lateral surface 311, a medial surface 314, a first A-P surface 313a, a second A-P surface 313b, a first proximal surface 315, a second proximal surface 316, and a distal surface 318. Additionally, trial proximal body may also have a second bore 322 bounded by a cutout 320 and a first bore 321 that intersects second bore 322. First and second bores 321, 322 are arranged within trial proximal body in a similar manner to that of implant proximal body 110.

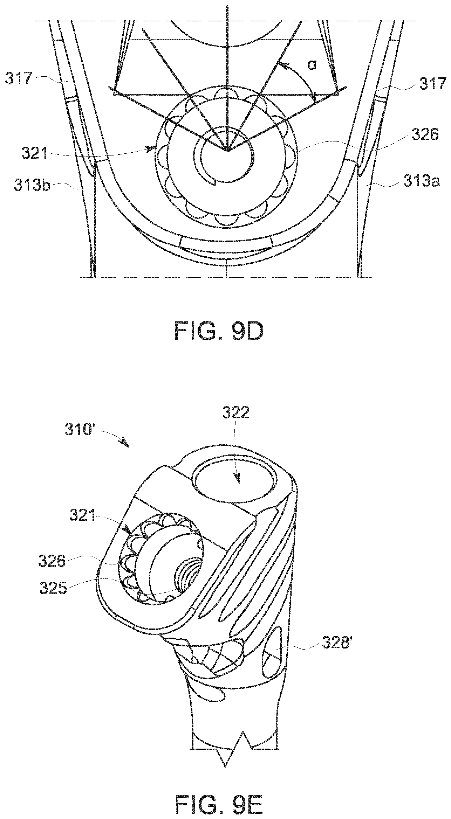

However, trial proximal body 310 differs from implant proximal body 110 in that first bore 321 may be a scalloped bore with a threaded opening 325 disposed therein, rather than a smooth bore like that of first bore 121 of implant proximal body 110. In this regard, scalloped bore 321 may include scallops 326 that are formed within trial proximal body 310 itself similar to scalloped opening 208 of proximal body trialing adapter 200. In this regard, first bore 321 may have a plurality of scallops or splines 326 arrayed about a central axis of first bore 321. For example, first bore 321 may have twelve scallops 326, as shown in FIG. 9D. In another example, first bore 321 may have seven scallops 326 like that of the proximal body trialing adapter 200 depicted in FIG. 3A. In further examples, first bore 321 may have more or fewer scallops 326, such as 3 to 12 scallops, for example. Furthermore, scallops 326 may be rotationally offset from each other by the same incremental angle α previously described. Thus, first bore 321 may be configured to clock a trial joint component, such as trial tray 220 or humeral head coupler trial 250, in various eccentricity configurations.

Although the articular trial components, such as insert 210, tray 220, insert 230, and coupler trial 250, were described above in relation to their use with respect to implant proximal body 110, it should be understood that their use is equally applicable with trial proximal body 310. However, because trial proximal body 310 has a scalloped opening 321 formed therein, proximal body trialing adapter 200 may not be utilized with trial proximal body 310.

Another difference between trial proximal body 310 and implant proximal body 110 is that, instead of porous patches 117, trial proximal body 310 may include visualization pockets 317. As shown, in FIGs. 9B and 9C, pockets 317 may be formed in trial proximal body 310 in the same shape and location as porous patches 317 on implant proximal body 110. Thus, during trailing, a surgeon can visualize where porous patches 117 would be located relative to anatomical structures, such as soft tissue, to help determine the viability of the particular humeral trial prosthesis 300 for implementation in the final humeral prosthesis 100.

A further difference between trial proximal body 310 and implant proximal body 110 is in its connection feature. As previously described, implant proximal body 110 may include a tapered post 119 that taper-locks with another structure, such as an implant spacer 150 or implant stem component 130. However, such connection feature is generally preferable for a permanent or semi-permanent connection which is most suitable for a final implant, such as implant proximal body 110. On the other hand, trial proximal body 310 may be a temporary structure that, desirably, is quickly assembled and disassembled during a surgical procedure. In this regard, trial proximal body 310 may instead include a quick connect/disconnect mechanism for connection to other components of humeral trial prosthesis. An exemplary quick connect/disconnect mechanism is shown in FIGs. 9B and may include a pair of outer legs 327 and a pair of inner legs 329.

Inner legs or inner flexures 329 are positioned adjacent to outer legs or outer flexures 327 and may each include inwardly extending protrusions 329a. Outer legs 327 may be parallel to inner legs 329 and may be offset from outer legs by a gap. Outer legs 327 are generally stiffer than inner legs 329 and, therefore, are generally more resistant to movement than inner legs 329 which are configured to move outwardly from a first position to a second position. Inner legs 329 are biased toward the first position. Therefore, outer legs 327 act as a limiter or a stop for inner legs 329 to prevent inner legs 329 from being moved too far and risking breakage or permanent deformation. As such, the outermost position inner legs 329 can assume is a position in which inner legs 329 abut outer legs 327. The gaps between outer and inner legs 327, 329 can provide some difficulties for cleaning and sterilization as debris can get caught up in such gaps. To help alleviate such difficulties, each outer leg 327 may include a cleaning slot 328 extending therethrough and communicating with the gap. This may make it easier to flush or otherwise remove debris from the gap not only during post-surgery cleaning and sterilization, but also during the procedure to ensure that inner legs 329 have freedom of movement.

FIG. 9E depicts an exemplary trial proximal body 310' according to another embodiment of the present disclosure. Trial proximal body 310' is similar to trial proximal body 310 in that it includes first and second bores 321, 322. Additionally, first bore 321 may be a scalloped bore, as previously described. However, trial proximal body 310' differs in that it is an implantable trial. In other words, in some procedures, such as a primary shoulder replacement or a revision shoulder replacement in which sufficient bone stock exists, proximal humerus 53 may not need to be removed, but instead may be prepared or otherwise shaped to accommodate a prosthesis that is inserted into the bone such that the bone of the proximal humerus 53 surrounds proximal body 310'. Trial proximal body 310' may be utilized in such a procedure and, therefore, is configured to be inserted into a void or cavity within a proximal end of a humerus 50. As such, its exterior geometry is generally slimmer than that of trial proximal body 310 so as to fit within the bone void. Additionally, proximal body 310' may have cleaning windows 328' extending therein which may facilitate cleaning and sterilization after each procedure.

FIGs. 9F-9H depict an exemplary trial spacer 350. Trial spacer 350 may share common features with implant stem component 130, and in particular, adapter 140. For instance, trial spacer 350 may include a body 354, divots 358, and a proximal collar or shoulders 359. In this regard, divots 358 are bounded at a proximal side thereof by shoulders 359 and at a distal side thereof by body 354. This allows features, such as inner legs 329 of trial proximal body 310, to securely engage to trial spacer 350. However, unlike adapter 140, trial spacer 350 may include a post 356 extending proximally from body 354. Post 356 is preferably cylindrical but for two flats 356a-b extending axially along post 356, the flats 356a-b preferably being parallel to one another. This configuration may prevent rotation of components that slide over post 356 and may also limit the possible rotational orientations of other components relative to post 356, which may help align different features among different components.

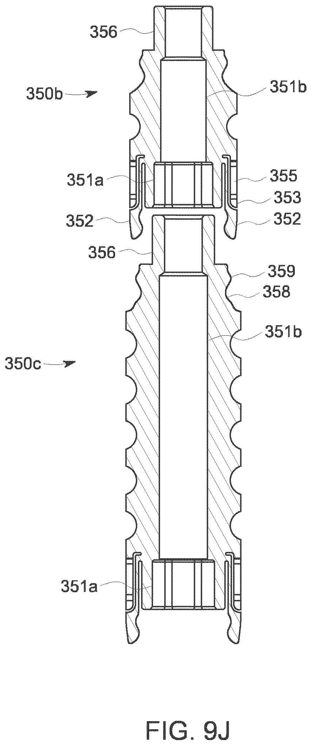

Additionally, a bore 351 may extend axially entirely through spacer 350 including post 356. Such bore 351 may have a first portion 351a and a second portion 351b, as shown in FIG. 9J which is a cross-sectional view of other spacers with the same bore features. First portion 351a of bore 351 may be located at a distal end of spacer 350 and may include opposing flats 357a-b, as illustrated in FIG. 9H, for engagement with a post 356 and corresponding flats 356a-b thereof of another spacer 350 or another component, such as taper protector insert 370 and expandable trial stem component 330, for example, each of which are described in more detail below. Second portion 351b may be substantially cylindrical and may be located toward a proximal end of spacer 350 extending through post 356. This allows a tool to traverse a length of spacer 350.

Trial spacer 350 may also share common features with trial proximal body 310. More specifically, trial spacer 350 may include inner legs 352 with an inwardly extending protrusion 352a, outer legs 353, and cleaning slots 355 just like that of trial proximal body 310 which allows trial spacer 350 to be connected to other trial spacers 350 and to expandable trial stem 330 (discussed below) or implant stem component 130 (discussed above), as previously described with respect to trial proximal body 310.

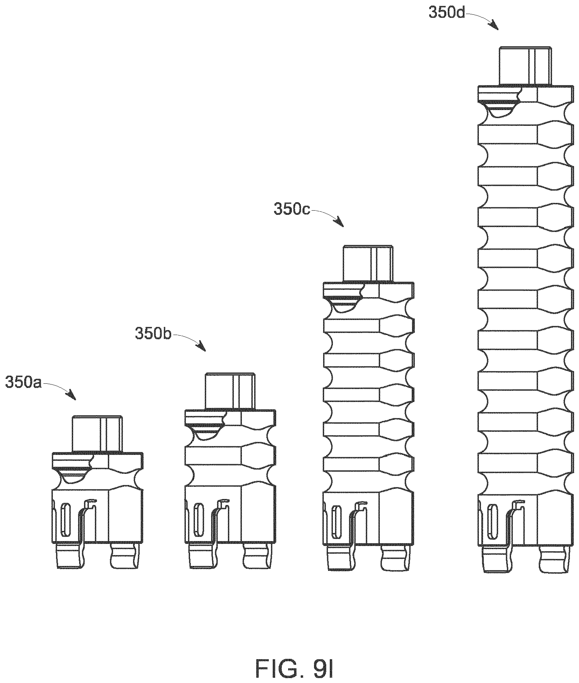

As with other components described herein, although a single trial spacer 350 is shown in FIGs. 9F-9H, it should be understood that a plurality of trial spacers 350 may be provided in a kit, of the same or differing heights. For example, FIG. 9I depicts a plurality of trial spacers of differing height that may be provided in a kit, such as a first spacer 350a, a second spacer 350b, a third spacer 350c, and a fourth spacer 350d. Each of these spacers 350a-d may have the same features as that described above with respect to trial spacer 350 with the exception that the spacer bodies thereof differ in height. For example, where the four trial spacers 350a-d are provided in a kit, such trial spacers may be provided with heights of 20mm, 30mm, 60mm, and 100mm, respectively, to accommodate a variety of clinical situations in the operating theater.

Trial spacers may be "push-to-connect." For example, as shown in FIG. 9J, when connecting a trial spacer 350b to trial spacer 350c, trial spacer 350b may be positioned adjacent to post 356 of spacer 350c with the flats 357a-b of first portion 351a bore 351 aligned with flats 356a-b of post 356. With this configuration, there may be only two rotational orientations in which trial spacer 350b may be coupled to trial spacer 350c. In either rotational configuration, as trial spacer 350b is advanced in the distal direction, each inner leg 352 aligns with a respective divot 358 of spacer 350c. Upon complete advancement of trial spacer 350b, trial spacer 350b is positioned relative to trial spacer 350c so that the inner legs 352, and particularly inwardly extending protrusions 352a, nest within the divots 358 of spacer 350c. Inner legs 352 and their inward protrusions 352a are shaped and positioned so that, as the trial spacer 350b is connected to trial spacer 350c, the shoulders 359 of trial spacer 350c first force inner legs 352 to flex outwardly. Then, when the inward protrusions 352a clear shoulders 359, inner legs 352 may "snap" back into place. This snap fit may provide audible and/or tactile feedback to confirm the connection. Although the inner legs may provide some resistance to trial spacer 350b being pulled proximally to disconnect from trial spacer 350c, because the trial spacers 350b-c are typically under compression during trialing, there is not a significant concern that trial spacer 350b would unintentionally disconnect from trial spacer 350c during use. In order to disconnect trial spacer 350b from trial spacer 350c, it merely needs to be pulled proximally away from trial spacer 350c. Further, as should be understood from the above description, the engagement between the flats 356a-b of 356 and the flats 357a-b of the internal cavity of trial spacer 350b prevent any rotation between the two components once connected.

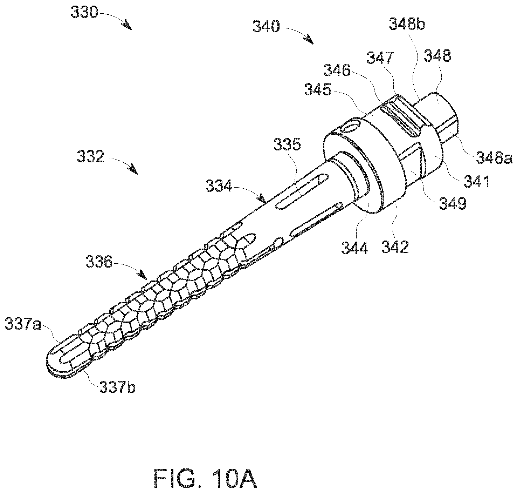

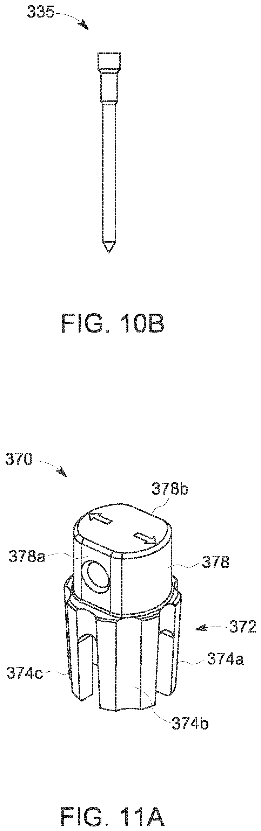

FIGs. 10A and 10B depict an exemplary expandable trial stem 330 in accordance with an embodiment of the present disclosure. Expandable trial stem 330 may include a stem 332, an adapter 340, and an expansion bolt 335. Stem 332 is configured to be inserted in an intramedullary canal of the remaining humeral shaft 52. However, unlike stem 132 of implant stem component 130, which may be impacted into bone in a press-fit manner, stem 332 may be inserted into humeral shaft 52 without impaction and thereafter expanded into secure engagement with bone. This may help preserve the bone and the shape of the intramedullary canal for later implantation of implant stem component 130.

Stem 332 may include a proximal portion 334 and a distal portion 336 that is split along its length to form first and second moveable stem portions 337a-b extending from and cantilevered to proximal portion 334. An expansion bolt or expansion driver 335 may extend through proximal portion 334 and may be moveable between first and second moveable stem portions 337a-b in a proximal-distal direction such that moving the expansion bolt 335 in a distal direction causes first and second stem portions 337a-b to move radially outwardly away from each other, and moving expansion bolt 335 in the proximal direction causes first and second stem portions 337a-b to move radially inwardly toward each other under their own bias. Thus, first and second stem portions 337a-b are biased toward a first configuration or contracted configuration and moveable to a second configuration or expanded configuration. While in the first configuration, stem 332 can be positioned within a bore in humerus 50 and freely rotated therein. While in the second configuration, first and second stem portions 337a-b press against the bone to secure it thereto until trialing is completed. Once trialing is completed, first and second stem portions 337a-b may be returned to the first configuration for removal by moving expansion bolt 335 proximally. This helps prevent damage and/or alteration to the bone prior to insertion of implant stem component 130. Further details of these operations and other exemplary stem and expansion bolt structures are set forth in U.S. Patent No. 9,011,549, the disclosure of which is incorporated by reference herein in its entirety.

Adapter 340 is similar to adapter 140 of implant stem component 130. For ease of review, like features are accorded like reference numerals to that of adapter 140. For instance, adapter 340 may include a proximal collar or shoulders 341, a distal collar 342, distal surface 344, adapter body 345, divots 346, protrusions 347, and flats 349. However, unlike adapter 140, adapter 340 may include a post 348 extending proximally from adapter body 345. Post 348 is preferably cylindrical but for two flats 348a-b extending axially along post 348, the flats 348a-b preferably being parallel to one another. This configuration may prevent rotation of components that slide over post 348 and may also limit the possible rotational orientations of other components relative to post 348, which may help align different features among different components. Post 348 may be configured to connect to one of trial spacers 350 and/or trial proximal body 310. Additionally, a bore (not shown) may extend axially entirely through adapter 340 including post 348. Expansion bolt 335 may be accessed through such bore.

In some embodiments, expandable trial stem 330 may not be used within trial humeral prosthesis. Instead, implant stem component 130 may be inserted into humeral shaft and used in conjunction with trial spacers 350 and trial proximal body 310 to trial a final implant configuration. As shown in FIG. 2H, implant adapter 140 may include a central bore 148 defined by a tapered interior surface, so that implant spacers (e.g., spacers 150a-d) with tapered posts 152 may be secured to the adapter 140 with a Morse taper fit. However, trial spacers 350a-d, as shown in FIGS. 9F-9J, may not include a post for engagement with adapter 140 and may instead be configured to engage adapter of 340 of expandable trial stem 330 and the non-tapered post 348 extending therefrom.

FIG. 11A depicts an exemplary taper protector insert 370 according to an embodiment of the present disclosure. Taper protector insert or trial alignment peg 370 may be used to help protect tapered bore 148 of implant stem component 130 during insertion thereof and to further adapt adapter 140 for connection with a trial spacer 350 or proximal body 310. Taper protector insert 370 generally includes a post 378 and a connection portion 372. Post or non-tapered portion 378 is similar to post 348 of expandable trial stem 330 in that it is generally cylindrical and includes opposed flats 378a-b. Connection portion or tapered portion 372 extends from post 378 and may include a plurality of flexures 374a-c , such as two to four flexures, for example, which together form an outer diameter that tapers inwardly in a proximal to distal direction and has a maximum diameter that is larger than an inner diameter of tapered bore 148 of adapter 148. Thus, when connection portion 372 is inserted into tapered bore 148 of implant stem component 130, the narrower bore 148 presses flexures 347a-c inwardly, which correspondingly press on tapered bore 148 outwardly to thereby secure taper protector 370 to adapter 140 of implant stem component 130. However, other embodiments of insert 370 may not include compliant flexures 374a-c but may rather have a rigid connection portion 372 which may be tapered and/or have a separate connection feature, such as a snap-fit feature, for connection to stem component 130.

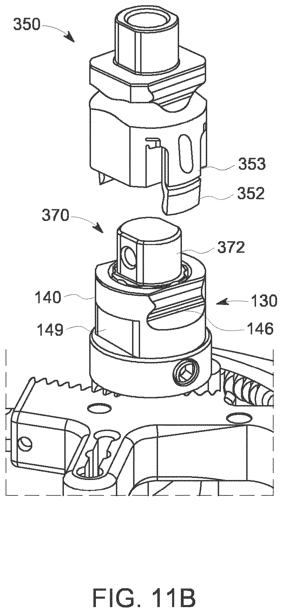

As shown in FIG. 11B, taper protector insert 370 may be inserted into tapered bore 148 of implant adapter 140 prior to coupling a trial spacer 350 or an impaction tool, such as inserter/extractor handle 700, discussed further below, to implant stem 110. Thus, tapered portion 372 of taper protector 370 may be received within the corresponding, but smaller, tapered bore 148 of implant stem component 130 such that the top, non-tapered portion 372 of taper protector 370 extends proximally therefrom. With this configuration, when trial spacer 350 is connected to adapter 140 such that inner legs 352 thereof engage divots 146 of adapter 140 and post 372 of taper protector 370 is received within the corresponding bore 351a in spacer to help stabilize and secure spacer 350 on adapter 140. Also, in this configuration, an impaction tool, such as inserter/extractor handle 700, may be separately connected to adapter 140 with taper protector 370 inserted therein. Taper protector 370 may help ensure there is not a large void space between the impaction tool and the implant adapter 140, helping to ensure that the impaction forces are evenly distributed and that the impaction tool does not damage implant adaptor 140 and tapered bore 148 therein.

Various instruments may be provided to help prepare humeral shaft 52 for receipt of the aforementioned prostheses 100, 300. Such instruments may include one or more of a bone clamp assembly 400, sounder 500, reamer planer 600, inserter/extractor handle 700, height gauge 800, impaction stand 900, and taper breaker tool 1000.

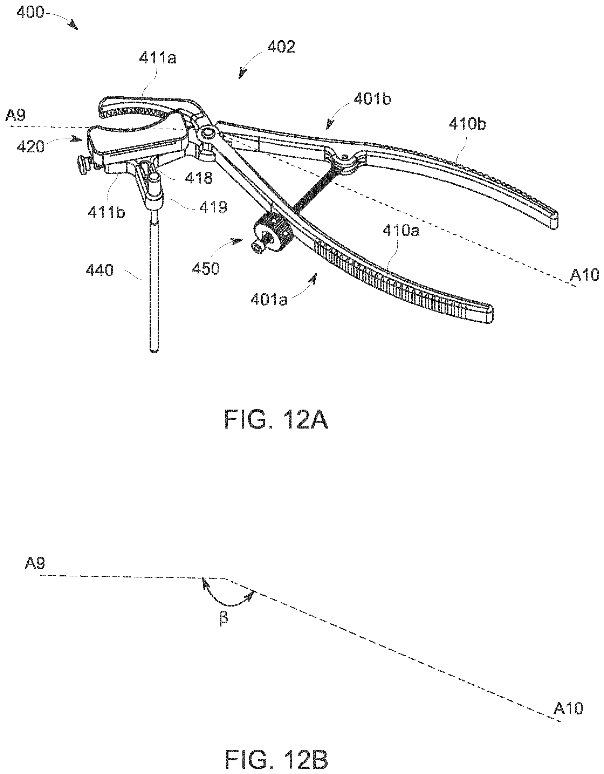

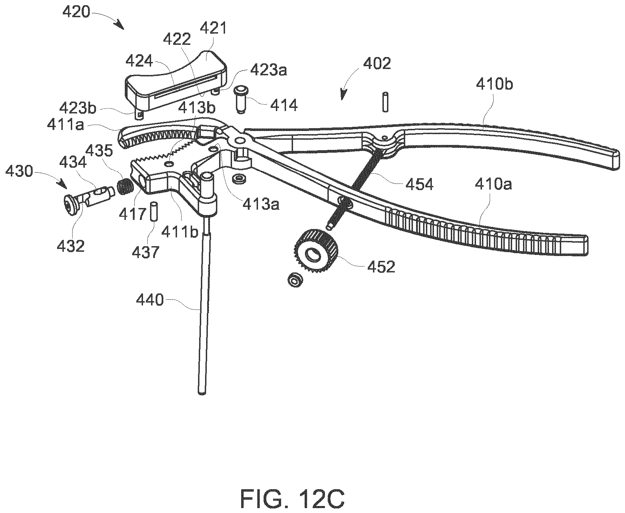

FIGs. 12A-12E depict an exemplary bone clamp assembly 400 according to an embodiment of the present disclosure. Bone clamp assembly 400 may include a bone clamp 402 and resection guide 420.

Bone clamp 402 may include a first member 401a and a second member 401b pivotably connected to first member 401a. Such connection may be made via a pin 414 which may define a pivot axis of about which first and second members 401a-b pivot relative to each other. First member 401a may include a first handle 410a and a first jaw 411a, and second member 401b may include a second handle 410b and a second jaw 411b. First and second members 401a-b cross each other at a location between the jaws 411a-b and handles 410a-b. The pivotable connection may be made at this location such that moving handles 401a-b closer together may move jaws 411a-b closer together, and moving handles 410a-b further apart may move jaws 411a-b further apart. However, other arrangements are contemplated in which handles 410a-b do not overlap and moving handles 410a-b further apart moves jaws 411a-b closer together, and moving handles 410a-b further apart moves jaws 411a-b closer together.

As shown in FIGs. 12A and 12B, a jaw axis A9 extends along the length of jaws 411a-b and is located equidistant between jaws 411a-b, and a handle axis A10 extends along the length of handles 410a-b and is located equidistant between handles 410a-b. Such axes A9, A10 may intersect each other at an angle β, as shown in FIG. 12B. This angle β may be an obtuse angle and may be 130 degrees, for example. In other embodiments, angle β may be 120 to 140 degrees. This angular arrangement between handles 410a-b and jaws 411a-b helps provide clearance for a bone saw to approach resection guide 420 and for positioning of soft tissue when jaws 411a-b are clamped onto humeral shaft 52.

Bone clamp 402 may also include a handle locking mechanism 450. Handle locking mechanism 450 can include a threaded shaft 454 extending from one of the first and second handles 410a-b and through the other of the first and second handles 410a-b. Additionally, a knob 452 may be threaded to threaded shaft 454 and positioned external to the handle 410a-b through the threaded shaft 454 extends. For example, as shown in FIG. 12A, threaded shaft 454 extends from second handle 410b and through first handle 410a. Knob 452 is threaded to threaded shaft 454 and positioned external to first handle 410a so that rotating knob 452 in a first direction prohibits first and second handles 410a-b from moving away from each other thereby effectively securing jaws 411a-b in a clamped engagement with bone. Conversely, rotating knob 452 in a second direction allows handles 410a-b to move away from each other thereby allowing jaws 411a-b to be disengaged from bone. In either a right-hand or left-hand configuration of clamp 402, knob 540 and threaded shaft 454 may be placed in an opposite direction. Other handle locking mechanisms are also contemplated, such as a ratchet mechanism, for example.

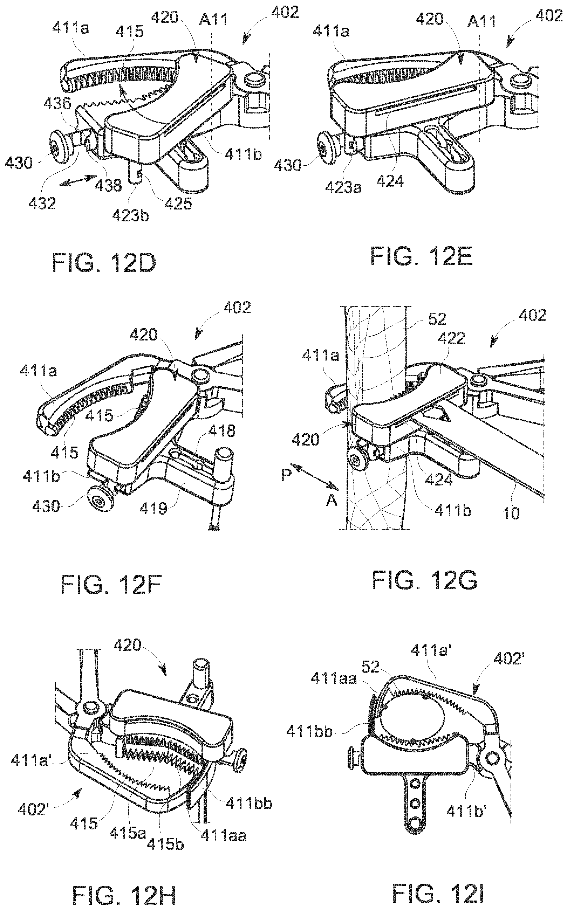

First jaw 411a may be referred to as a posterior jaw as it may be positioned along a posterior aspect of humeral shaft 52 when clamped thereto, and second jaw 411b may be referred to as an anterior jaw is it may be positioned along an anterior aspect of humeral shaft 52 when clamped thereto, as shown in FIG. 12G. First and second jaws 411a-b may each have a first row of teeth 415 which may help prevent slippage of jaws 411a-b when engaged to bone. However, in other embodiments, second jaw 411b may have first and second rows of teeth 415a-b, as shown in FIG. 12H, which may facilitate perpendicular positioning for accurate perpendicular resection, as explained further below.

Second jaw 411b may be configured to connect to resection guide 420. In this regard, second jaw 411b may include first and second holes 413a-b extending through a superior surface thereof. A transverse opening 417 may also extend into second jaw 411b through a free end thereof and transverse to first and second holes 413a-b and such that transverse opening intersects second hole 413b. Second jaw 411b may also include a plunger 430, a spring 435, and a pin 437. Plunger 430 may include a first slot 434 which may extend through plunger in an S-I direction. Plunger 430 may also include a second slot or captured slot 432 extending into a side of plunger 430 offset longitudinally from elongate slot 434 and in an anterior to posterior direction. A protrusion 436 may extend inwardly into second slot 432 and longitudinally into an aperture defining an entrance to second slot 432, and a cam surface 438 may be formed on protrusion 436 adjacent to second slot 432, as best shown in FIG. 12D. When assembled, plunger 430 and spring 435 may be positioned within transverse opening 417 such that spring 435 biases plunger 430 in an outward direction toward the free end of second jaw 411b. Plunger 430 may be moveably connected to second jaw 411b via pin 437 and first slot 434. In this regard, when plunger 430 is received within transverse opening 417, first slot 434 aligns with second hole 413b in second jaw 411b, and pin 437 extends through second hole 413b into engagement with first slot 434. This keeps plunger 430 secured within transverse opening 417, while allowing it to slide back and forth within transverse opening 417 from a first position to a second position. Thus, plunger 430 may be biased to the first position and pushing on plunger 430 may move plunger to the second position against the bias of spring 435.

Second jaw 411b may also have a drop rod protrusion extending from an exterior or anterior side thereof. Such drop rod protrusion 419 extending outwardly and anteriorly therefrom. Drop rod protrusion 419 may have a rod slot 418 and/or one or more holes extending therethrough such that a drop rod, such as drop rod 440 shown in FIG. 12A, may be placed through rod slot 418 in order to align jaws 411a-b relative to a shaft axis of humeral shaft 52.

Resection guide 420 may be modular such that it can be connected to bone clamp assembly 400, and, in the particular embodiment depicted, to second jaw 411b. Resection guide 420 may include a first surface or superior surface 421 and a second surface or inferior surface 422. Superior surface 421 may be planar and may form an uncaptured resection guide surface. Resection guide 420 may also include a resection guide slot 424 which is completely enclosed to form a captured resection guide slot. Such resection guide slot 424 is located between superior and inferior surfaces 421, 422 and may be a predetermined distance from superior surface 421. This allows a surgeon to resect along superior resection surface 421 and then, if desired, resect through resection guide slot 424 to remove a known amount of bone relative to that of the first resection. For example, cutting through resection guide slot 424 may remove an additional 2mm of bone. This arrangement also allows a surgeon to know how much bone remains above first and second jaws 411a-b after resection as it may be desirable to have a known quantity of bone above such jaws 411a-b for additional operations subsequent to resection, such as reaming operations, for example. In this regard, resecting along superior surface 421 may leave about 10mm of bone above first and second jaws 411a-b, while resecting through captured guide slot 424 may leave about 8mm of bone above first and second jaws 411a-b, for example.

Resection guide 420 may also include first and second posts 423a-b extending from inferior surface 422. Second post 423b may have an indentation or notch 425 extending within it transverse to a longitudinal axis of post 423b, as best shown in FIG. 12D. First post 423a is configured to be received within first opening 413a in second jaw 411b. Second post 423b is configured to be received with in second slot 432 of plunger 430, and protrusion 436 of plunger 430 is configured to engage notch 425, as described in more detail below.

FIGs. 12D and 12E illustrate the connection of resection guide 420 to second jaw 411b. As shown, first post 423a may be inserted into first opening 413a of second jaw 411b which forms a rotational axis A11 about which the remainder of resection guide 420 rotates or swings into final position. In this regard, the end of resection guide 420 with second post 423b may be rotated toward plunger 430 which protrudes out of the free end of second jaw 411b so that second slot 432 faces toward second post 423b. When second post 423b comes into engagement with plunger 430, second post 423b contacts cam surface 438 which causes plunger 430 to be pushed inwardly to the second position against the bias of spring 435. This allows second post 423b to move deeper within second slot 432. Once second post 423b passes this cam surface 438 and is fully positioned within second slot 432, the bias of spring 435 causes plunger 430 to move back to the first position. As this occurs, protrusion 438 engages notch 425 in second post 423b and blocks second post 423b from moving back along the same rotational path it just took. Thus, in this arrangement, resection guide 420 is prohibited from rotating back out from engagement with plunger 430 unless plunger 430 is pushed back into the second position. Additionally, the engagement between protrusion 426 and notch 425 prevents resection guide 420 from being lifted upward and away from second jaw 411b. Connection of resection guide 420 to second jaw 411b may be made before or after bone clamp 402 is clamped to a bone.

Bone clamp assembly 400 may be universal in that it may be used on both a left and a right humerus. For example, bone clamp assembly 400 as shown in FIG. 12A is arranged for clamping a left humerus of a patient such that first jaw 411a engages a posterior aspect of the left humerus, second jaw 411b engages an anterior aspect of the left humerus and handles 410a-b extend laterally away from the patient's body. When used in a right humerus, clamp 402 may be flipped over so that first and second jaws 411a-b engage the posterior and anterior aspect of the right humerus, respectively, and handles 410a-b extend laterally away from the patient's body. Second jaw 411b may be configured to allow resection guide 420 to be connected to opposite sides thereof. In this regard, first hole 413a may extend entirely through jaw 411b so that first post 423a of resection guide 420 can engage first hole 413a from either side of second jaw 411b. Additionally, plunger is constructed so that second post 423b can engage plunger from either side of second jaw 411b.

With resection guide 420 connected to second jaw 411b and when bone clamp 402 is clamped to humeral shaft 52, resection guide slot 424 is preferably aligned perpendicular to a shaft axis of humeral shaft 52. Such perpendicularity may be assisted by the arrangement of teeth 415 formed on first and second jaws 411a-b. In this regard, a typical humeral shaft may have a triangular cross-sectional shape. As mentioned above, each jaw 411a-b may have one or more rows of teeth 415 which may help orient jaws 411a-b and resection guide 420 into the desired perpendicular position. For example, first jaw 411a may have a one row of teeth 415 that may extend along an arcuate path, as shown in FIG. 12F. On the other hand, second jaw 411b may have a first row of teeth 415a and a second parallel row of teeth 415b that may each extend linearly in a general "V" shape pattern, as shown in FIG. 12H. In operation, second jaw 411b may be placed against an anterior face of humeral shaft 52, while first jaw 411a may be positioned against a posterior aspect of humeral shaft 52, as shown in FIG. 12G. The arcuate extension of teeth 415 on first jaw 411a, and the V-shape extension of the first and second rows of teeth 415a-b of second jaw 411b interact with the triangular structure of humeral shaft 52 such that jaws 411a-b are urged into a perpendicular alignment if they are not already perpendicularly aligned. Furthermore, the additional second row of teeth 415b of second jaw 411b may help prevent clamp 402 from pivoting out of perpendicular alignment. In other words, second row of teeth 415b helps constrain clamp 402 from pivoting as compared to a second jaw 411b with only first row of teeth 415a. Perpendicular alignment can be verified by drop rod 440 extending through rod slot 418 in second jaw 411b. Once properly aligned, a bone saw, such as reciprocating saw 10 of FIG. 12G, may be passed through captured slot 424 or along uncaptured superior guide surface 422 to resect humeral shaft 52. Once the bone is resected, resection guide 420 can be removed by pressing on plunger 430 to release second post 423b, swinging second post 423b out of second slot 432, and pulling up on resection guide 420 to remove first post 423a from first opening 413a. Clamp 402 may remain attached to the bone 52 for further operations and may facilitate easy manipulation of the bone via clamp 402 throughout the remainder of the procedure.

FIGs. 12H and 12I depict alternative bone clamp jaws 411a', 411b'. First and second jaws 411a', 411b' are the same as jaws 411a-b with the exception that each jaw 411a', 411b' includes a jaw extension 411ab, 411bb extending from a respective free end thereof. As shown in FIG. 12I, when first and second jaws 411a', 411b' are clamped onto humeral shaft 52, first and second jaw extensions 411aa-bb, come together and overlap such that first and second jaws 411a', 411b' completely encompass a circumference of humeral shaft 52. In addition, at least one of the first and second jaw extensions 41 1aa-bb may contact the bone 52 as a third point of contact. Thus, teeth 415 of first jaw 411a' may form a first point or line of contact, teeth 415a-b of second jaw 411b' may form a second point or line of contact, and at least one of jaw extensions 411aa-bb may form a third point or line of contact. This embodiment may be particularly beneficial for slippery bone. In some cases, bone clamp 402' may rotate, tilt, or otherwise adjust itself due to gravity on slippery bone causing bone clamp 402' to come out of alignment with the bone 52. The closed ends of jaws 411a', 411b', which are closed due to jaw extensions 41 1aa-bb, help to locate three points of contact in the same plane to help maintain the position of bone clamp 402' over slippery bone.

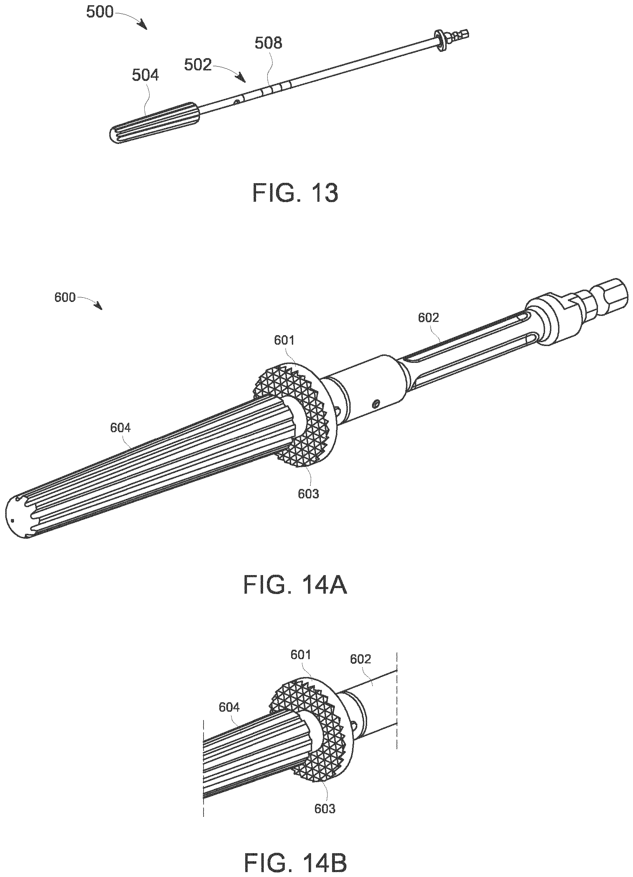

FIG. 13 depicts an exemplary sounder 500 according to an embodiment of the present disclosure. Sounder or reamer 500 may be used to help shape an intramedullary canal of humeral shaft 52 for reception of implant stem component 130 and expandable trial stem 330. Sounder 500 may include an elongate shaft 502 and a reamer head 504 at a distal end of shaft 502. Reamer head 504 may have a conical cutting profile configured to form a conical bore within humeral shaft 52. Additionally, shaft 502 may have depth markings 508 positioned along shaft 502 to indicate depth of cutting head 504 within bone.

FIGs. 14A and 14B depict an exemplary reamer planer 600 according to an embodiment of the present disclosure. Reamer planer 600 may also be used to help shape the intramedullary canal of humeral shaft 52 and to form a planar surface at a proximal end of humeral shaft 52 that is perpendicular to the intramedullary canal thereof. Reamer planer 600 may include an elongate shaft 602, a reamer head 604, and a planeing member 604. Reamer head 604 may have a conical cutting profile configured to form a conical bore within humeral shaft 52. Reamer planer 600 may be implemented after sounder 500. In this regard, reamer head 604 may have a larger diameter than sounder 500 such that reamer head 604 provides the final shape of the bore within humeral shaft 52. Planeing member or planeing plate 601 may be positioned between elongate shaft 602 and reamer head 604. Planeing plate 601 may extend radially outwardly and may include a planeing surface 603 oriented perpendicular to a longitudinal axis of reamer head 604. Planeing surface 603 may include cutting features thereon which are configured to shape a proximal end of the resected bone 52 so that it forms a planar surface and a bloody bone platform 144 that facilitates bone growth into the porous distal surface 144 of implant stem component 130.

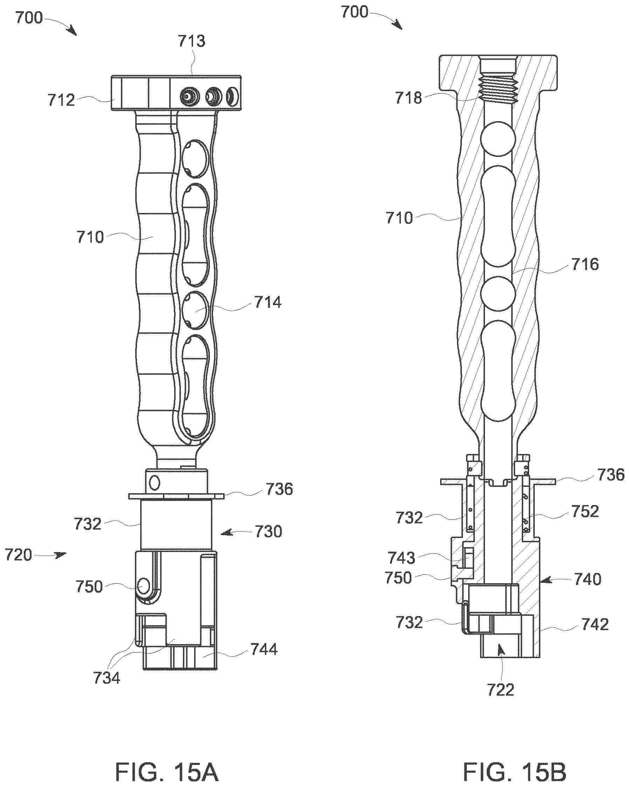

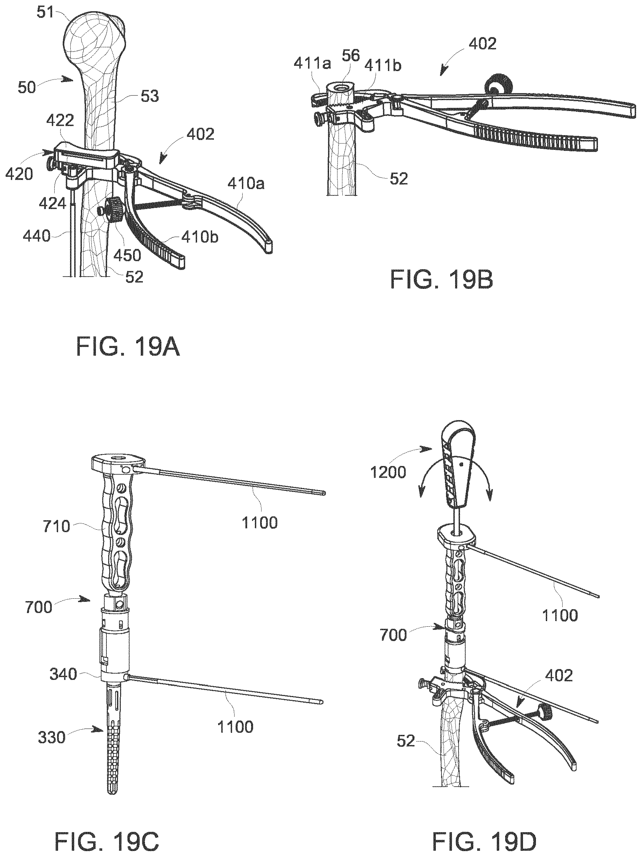

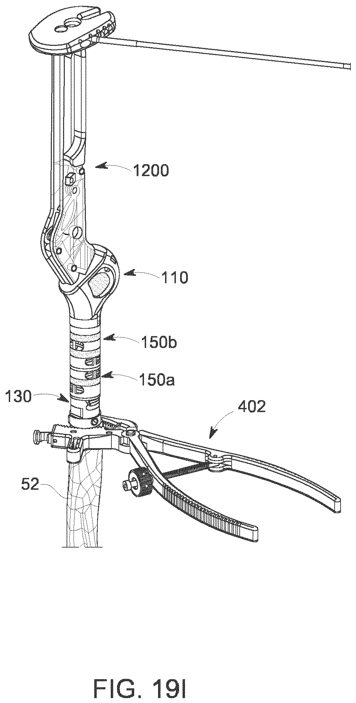

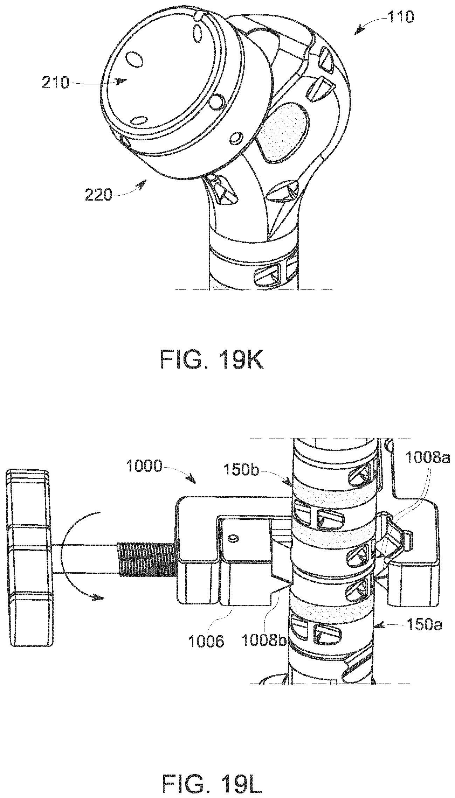

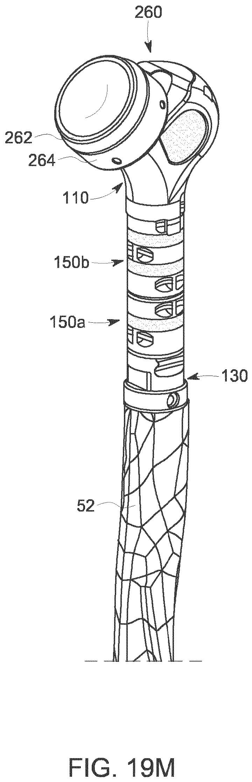

FIGs. 15A-15F depict an exemplary inserter/extractor handle assembly 700 according to an embodiment of the present disclosure. Handle assembly 700 generally includes a handle 710 and a connector 720 disposed at a distal end of handle 710. Handle 720 may include a collar 712 at a proximal end which may have a cross-sectional dimension greater than the remainder of handle 710 and may define an impaction surface 713 for impacting handle 700 and any device connected thereto. Handle 710 may also include contouring along its length to assist with easier gripping and one or more through holes 714 to reduce the weight of handle 710. Also, as shown in FIG. 15B, an axial bore 716 may extend entirely through handle 710 and connector 720 which may allow a driver, such as the driver 1200 shown in FIG. 19D, to be extended through handle assembly 700 to engage a device connected thereto. Such device can be implant stem component 130 or expandable stem trial 330, for example. Axial bore 716 may also have a threaded portion 718 for threaded engagement with a slap-hammer, for example, to assist with extraction of such implantable device.

Connector 720 generally includes a first portion 740 connected to a distal end of handle 710 and a second portion 730 moveable relative to first portion 740 from a first position, in which an adapter can engage first portion 740, and a second position, in which second portion 730 also engages the adapter and secures the adapter to handle assembly 700. Such adapter can be any of the aforementioned adapters, such as adapter 140 of implant stem component 130, with or without taper protector insert, or adapter 340 of expandable trial stem 330.

An exemplary connector 720 is shown in FIGs. 15A and 15B and may include a connector body or first portion 740. Connector body 740 may be connected to a distal end of handle 710 and may extend distally therefrom. Connector body 740 may be integrally connected to handle 710 such that connector body 740 and handle 710 form a monolithic structure, or connector body 740 and handle 710 may be separately formed and modularly connected, such as via a pin, thread, taper fit, or the like. Connector body 740 may include a downwardly extending wall or flange 742 which may be disposed at one side of handle assembly 710 and offset from a longitudinal axis thereof. Connector body 740 may also include a pair of arms 744 that extend radially inwardly and that may be each configured to engage a corresponding flat on an adapter, such as flats 149 and 349. Arms 744 may also each define a recess or slots 746 at respective proximal sides thereof. Each of such recess 746 may be configured to receive a shoulders, such as shoulders 141 and 341.

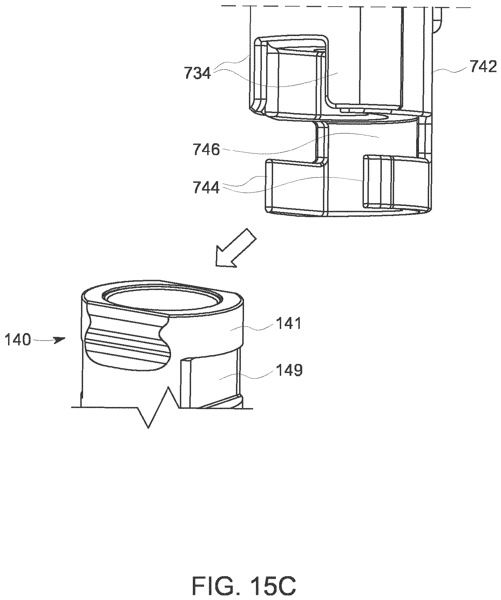

Connector 720 may also include a sleeve or second portion 730. Sleeve 730 may include a cylindrical main body 732 which may be hollow and sized to overlie at least a portion of connector body 740. Sleeve 730 may also be moveable relative to connector body 740 in a proximal distal direction between a first position and a second position. In this regard, sleeve 730 may be connected to main body 740 via a pin 750, which may extend through sleeve 730 and into an axial slot 743 of main body 740, as shown in FIG. 15B. A spring 752 may be connected to connector body 740 and to sleeve 730, as also shown in FIG. 15B. Spring 752 may be a compression spring that biases sleeve 730 toward the first position. Sleeve 730 may also include a proximal flange 736 which may extend circumferentially around main body 740 at a proximal end thereof which may allow a surgeon to pull up on sleeve 730 against the bias of spring 752 to move sleeve 730 from the first position to the second position. Sleeve 730 may also include one or more protrusions 734 extending from main body 732 in a distal direction and may be positioned at an opposite side of the longitudinal axis of handle assembly 700 as arms 744 of connector body 740, as best shown in FIG. 15C. In this regard, the downwardly extending wall 742 of connector body 740 and protrusions 734 of sleeve 730, when sleeve 730 is in the first position, may together define a cavity 722 configured to receive one of the aforementioned adapters 140, 340.

FIGs. 15C-15E illustrate an operation of coupling handle to adapter 140 of implant stem component 130. Although adapter 140 is shown without taper protector insert 370, it should be understood that the procedure for coupling handle assembly 700 to adapter 140 may be the same when taper protector insert 370 is disposed within tapered bore 148 of adapter 140. Additionally, the procedure is the same when connecting to adapter 340 of expandable trial stem 330. As shown in FIG. 15C, prior to coupling handle 700 to adapter 140, connector 720 is prepared to receive adapter 140. In this regard, sleeve 730 may be pulled proximally against the bias of spring 752 and so that the proximal force on sleeve 730 is maintained to keep sleeve 730 in this proximal or unlocked position. In this proximal or unlocked position, arms 744 of connector body 740 are exposed, as are recesses 746 proximal to arms 744. Arms 744 may be sized and shaped to contact the corresponding flats 149 on adapter 140, while recesses 746 may be sized and shaped to receive shoulders 141 of adapter 140.

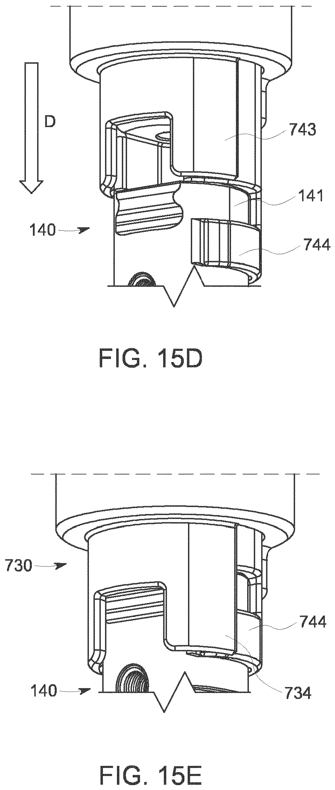

As shown in FIG. 15D, handle assembly 700 is then slid laterally so that arms 744 are slid laterally over flats 149 while sleeve 730 is maintained in the proximal or unlocked position. At this point, the user may simply release the force on the sleeve 730, so that the spring 752 decompresses, forcing sleeve 730 to slide in the distal direction D.

FIG. 15E illustrates sleeve 730 having transitioned back to the second or locked position. In this locked position, each protrusion 734 overlies a portion of main body 145 of adapter 140 as well as a portion of a corresponding arm 744. In this locked position, which is maintained by spring 752 in the absence of applied forces, lateral, axial, and rotational movement between adapter 140 and handle assembly 700 is prevented.

It should be understood that handle assembly 700 is merely exemplary of a handle assembly that may be used to connect to an adapter, such as adapters 140 and 340. Other inserter/extractor handles with other connector mechanisms may also be utilized, examples of which are described in the heretofore referenced '216 Application, incorporated by reference herein.

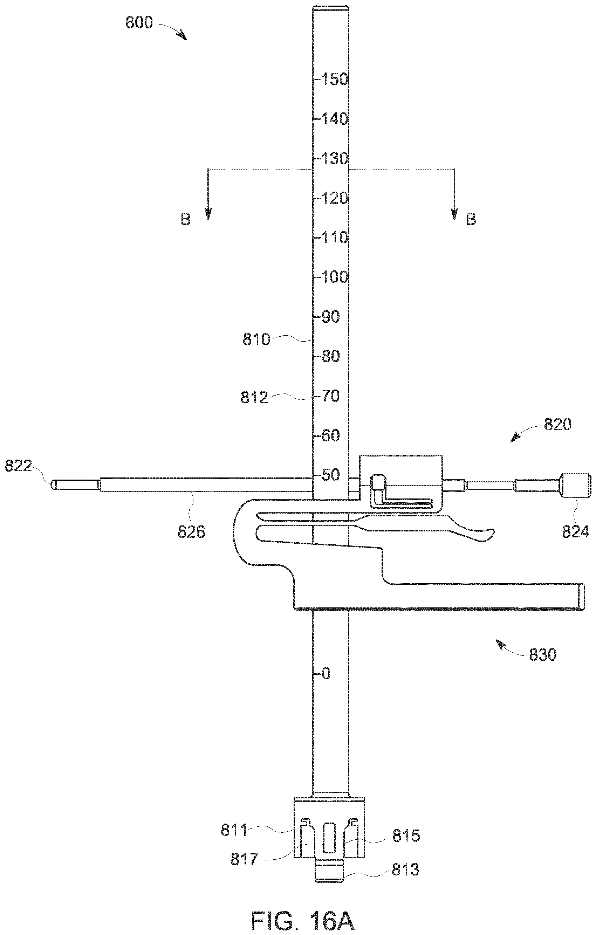

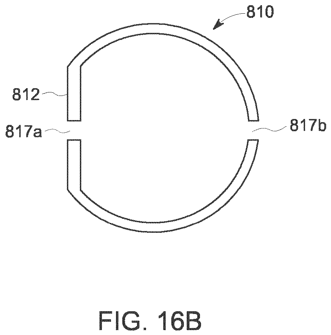

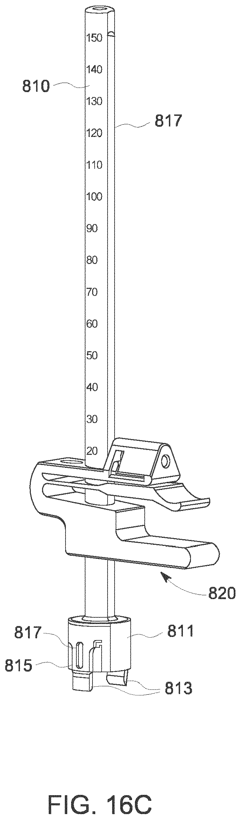

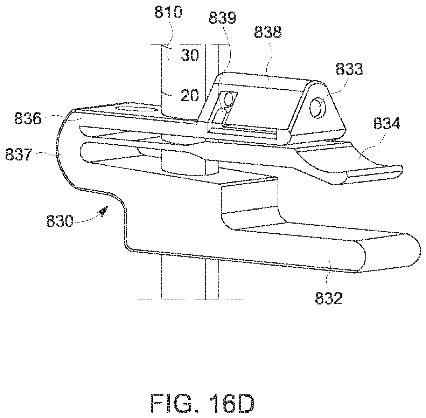

FIGs. 16A to 16D depict an exemplary height measuring gauge 800 according to an embodiment of the present disclosure. Height measuring gauge 800 may include a main body 810, a pointer 820, and a clamp 830. Main body or scale 810 may be an elongate shaft with a generally symmetrical cylindrical shape to facilitate rotation of clamp 830 about scale 810. However, in some embodiments a face 812 of scale may be flattened, such as to form a "D" cross-sectional shape, as shown in FIG. 16D, for example. Such flattened face 812 may be positioned on a side of scale 810 that may face a patient's glenoid and may help prevent rotation of clamp 820 about scale 810. Scale 810 may include indicia 840 provided thereon (*e.g.,* hash marks with millimeter markings) and may include a slot 817 (which may be enclosed on one or both axial ends) extending along an axial length of scale 810. Slot 817 may define two openings 817a-b in a perimeter of scale 810 positioned about 180 degrees apart which facilitates passage of pointer 830 therethrough and axial sliding thereof.

Scale 810 may have a mating member or connector 811 at a distal end thereof configured to couple to an adapter, such as adapter 140 and adapter 340 of implant stem component 130 and expandable trial component 330, respectively. In this regard, mating member 811 may include features similar to that of trial proximal body 810 and trial spacers 850. More specifically mating member 811 may include inner legs or flexures 813, outer legs or flexures 817, and cleaning slots 817 extending through outer legs 815. Inner legs 813 may be configured to mate with shoulders and divots, such as shoulders 141, 341 and divots 146, 346, of the respective adapter 140, 340. However, it should be understood that this is merely exemplary, and any suitable coupling mechanism may be used to couple scale 810 to expandable trial stem 330 or implant stem 130.

Pointer 820 and clamp 830 may together form an indicator member of height measuring gauge 800. Pointer or stylus 830 may be a general pin-shaped member, with a point leading end 822 intended to point to and/or contact a center of the patient's glenoid 58, and a trailing end 824 that has a slightly larger diameter than a main shaft 826 of pointer 820. Main shaft 826 of pointer 820 may pass through slot 817 of scale 810. In some embodiments, the enlarged trailing end 824 of pointer 820 may have a diameter that is larger than a width of slot 817 to ensure that trailing end 824 of pointer 820 does not pass through slot 817. This relationship is not necessary, however, and any suitable features (including an enlarged diameter) may be provided at the trailing end 824 of pointer 820 to facilitate a user gripping and moving pointer 820.

Clamp 830 may include an aperture through which scale 810 extends, a slider 832, and an arm 834 spaced from slider 832. Slider 832 may include a generally "D"-shaped aperture that receives the "D"-shaped scale 810 therethrough. Arm 834 may positioned proximal but otherwise aligned with slider 832 and may be cantilevered to a proximal extension 837 of slider 832 such that arm 834 may be depressed toward slider 832. Arm 834 may include a generally "D"-shaped aperture that receives scale 810, with apertures of slider 832 and arm 834 being generally aligned. However, the "D"-shaped aperture of arm 834 may be slightly angled so that, when there is no force applied to arm 834 relative to slider 832, the shape of the aperture of arm 834 maintains clamp 830 at the current height. In order to slide clamp 830 proximally or distally relative to scale 810, arm 834 may be depressed toward slider 832, causing the angled hole of arm 834 to change orientation relative to scale 810 and allow sliding of clamp 830 proximally or distally relative to scale 810. As soon as the force on arm 834 is released, it returns to its original orientation to fix clamp 830 at the current height. Main shaft 826 of pointer 820 may be used to read the indicia 812 on scale corresponding with main shaft 826 to determine an indicated height for proximal humeral prosthesis 100, as best shown in FIG. 16A.

Clamp 830 may also include proximal extension 836 and a receiver 838 disposed on proximal extension 836. Proximal extension 836 may be positioned proximal to arm 834 and may also extend from proximal extension 837 of slider 832. Proximal extension 836 may also have a "D"-shaped aperture to receive scale 810 therethrough similar to slider 832. Receiver 838 may be a generally triangular shaped structure located on proximal extension 836 and may define a through-hole 833 to receive pointer 820. Receiver 838 may hold pointer 820 in place by friction. For example, receiver 838 may include a flexure finger 839 that terminates in an upwardly projecting protrusion that presses against pointer 820 when pointer 820 is received the receiver 838. The protrusion provides a baseline level of friction against pointer 820 via flexure finger 839 so that pointer 820 will tend to remain stationary in the absence of applied force (other than the force applied by the flexure finger 839). A user may move pointer 820 by pushing or pulling pointer 820, with the pushing or pulling force overcoming the friction from the protrusion of flexure finger 839.

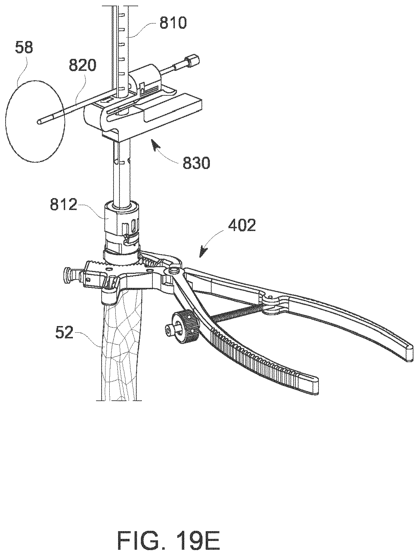

In use, after implanting expandable trial stem 310, or implant stem component 110, into humeral shaft 52, measuring gauge 800, and specifically mating member 811 of scale 810, may be pressed onto adapter 340 in the same way described in connection with trial spacer 350. At this point, pointer 820 may be, but is preferably not, coupled to clamp 830. With scale 810 coupled to trial stem 310, the user may depress arm 834 toward slider 832 and, while depressed, slide clamp 830 to a position (height and/or angle) that is near the patient's glenoid 58, as shown in FIG. 19E. If not already attached, pointer 820 may be inserted into through-hole 833 of receiver 838, and the position of pointer 820 may be fine-tuned or otherwise adjusted until it points to the center of the patient's glenoid 58. At this point, arm 834 may be released to temporarily re-lock clamp 830 in its position, and the height or distance measurement may be read by comparing the position main shaft 826 of pointer 820 to indicia 812 on scale 810. This distance may be used for any desired purpose, including, for example, to get a better idea of what combination of number and heights of trial spacers 350 and/or implant spacers 150 should be used to build up trial humeral prosthesis 300 or humeral prosthesis 100, respectively, minimizing the amount of trial and error necessary, and thus reducing surgery time and increasing patient outcomes.

It should be understood that height gauge 800 is merely exemplary of a height gauge that may be used in a shoulder replacement procedure to determine a height of humeral prosthesis 100. Other height gauges may also be utilized, examples of which are described in the heretofore referenced '216 Application, incorporated by reference herein.

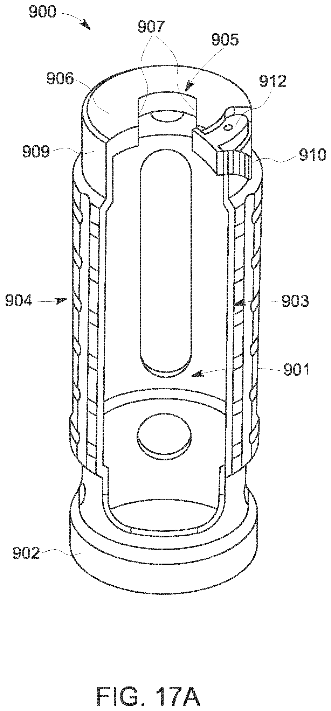

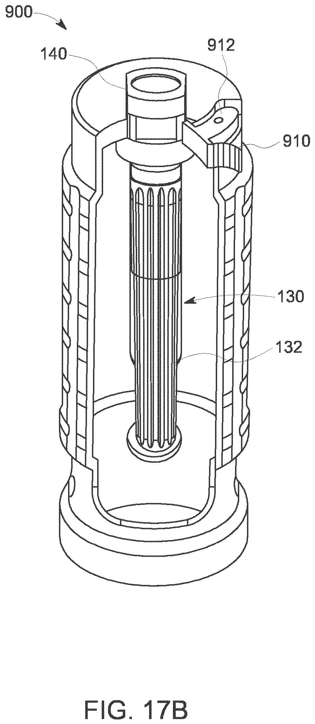

FIGs. 17A and 17B depict an exemplary impaction stand 900 according to an embodiment of the present disclosure. As described above, proximal body 110, implant stem 130, and spacers 150 may be connected together by a series of Morse taper locks to form humeral prosthesis 100. This may require that such components be impacted together to fully seat the taper locks and secure the components relative to each other. Impaction stand 900 may be used in the operating theater to help assemble humeral prosthesis 100. Impaction stand 900 may generally be a hollow cylindrical structure that may include a base 902 and an elongate body 904 extending from the base 902. However, other shapes are contemplated, such as a rectangular shape, for example.

Body 904 extends along a longitudinal axis and terminates at base 902 at a distal end and with an end cap or end plate 906 at a proximal end. Base 902 may have a diameter or cross-sectional dimension larger than a diameter or maximum cross-sectional dimension of body 904 so as to provide stability and support to body 904 during impaction. Body 904 includes a sidewall 909 that defines a cavity 901 configured to receive implant stem component 130 therein. In this regard, a side slot 903 may extend through sidewall 909 and into end plate 906 such that side slot 903 is in communication with cavity 901 and so as to form a notch 905 in end plate 906. Such notch 905 may be defined by opposing rails 907 that may extend parallel to each other, as shown in FIG. 17A, and may be spaced at a distance configured to engage flats 149 of implant stem adapter 140, as shown in FIG. 17B. A locking arm 910 may be connected to an end of one of rails 907. A cam or sliding member 912 may be connected to locking arm 912 and may be configured to slide along end plate 906 so as to move locking arm 910 between a first position and a second position.

In operation, impaction stand 900 may be placed on a back-table in an operating theatre such that base 902 is placed onto the table. Implant stem 130 may be inserted into cavity 901 through side slot 903 so that rails 907 engage flats 149 of stem adapter 140 to hold and secure stem component 130 in suspension, as shown in FIG. 17B. As adapter 140 is inserted into notch 905 between rails 907, adapter 140 engages locking arm 910 so that locking arm 910 moves from the first, unlocked position to the second, locked position, as also shown in FIG. 17B. Sliding member 912 helps hold locking arm 910 in this position, and arm 910 helps hold stem component 130 stationary within notch 905 as humeral prosthesis 100 is assembled thereto. In this regard, spacers 150 may be impacted to stem component 130, and proximal body 110 may be impacted to the last spacer 150 in the stack. As such, impaction stand 900 helps provide a stable platform for the assembly of humeral prosthesis 100.

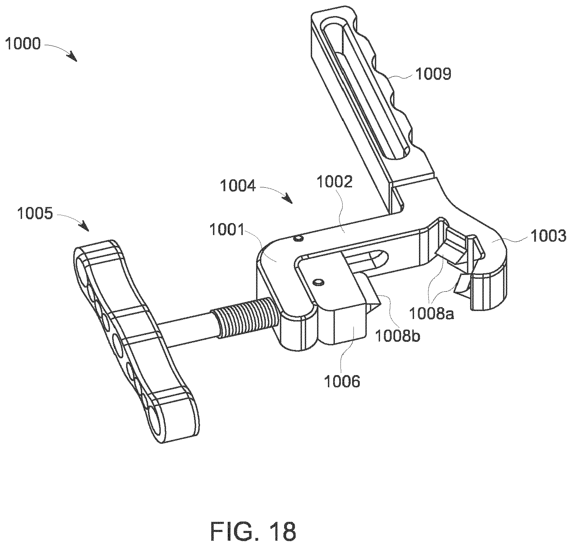

FIG. 18 depicts an exemplary taper breaker tool 1000 according to an embodiment of the present disclosure. After assembling humeral prosthesis 100 and trialing the same, it may be desirable to remove and/or replace an implant spacer 150 in favor of another spacer 150 of different length. In this regard, taper breaker tool 1000 may be configured to disconnect spacers 150 from each other, a spacer 150 from a stem component 130, and/or a spacer 150 from proximal body 110. As shown, taper breaker tool 1000 may include a stationary block 1004, a drive member 1005, a moveable block 1006, and a handle 1009. Stationary block 1004 may include a first member 1001, a second member 1002, and a third member 1003 arranged in a U-shape. Third member 1003 may include a first wedge 1008a extending in a direction toward first member 1001. Moveable block 1006 may also include a second wedge 1008b and may be disposed adjacent to first member 1001 such that second wedge 1008b of moveable block 1006 faces first wedge 1008a of third member 1003. Drive member 1005 may extend through first member 1001 and may be connected to moveable block 1006 so as to drive moveable block 1006 toward first wedge 1008a of third member 1003. In this regard, drive member 1005 may be threaded to first member 1001 to facilitate a driving action that allows wedges 1008a-b to pry the taper locked components apart, as illustrated in FIG. 19L. Handle 1009 may extend from first and/or second member 1002, 1003 to facilitate manipulation of taper breaker tool 1000.

The above-described implants, trials, and instruments may be provided as a system. In this regard, a humeral replacement system may include humeral prosthesis 100 including all components thereof, trial humeral prosthesis 300 and all components thereof, reverse trial and humeral head trial components 200, 210, 220, 230, 250, and instruments, such as bone clamp assembly 400, sounder 500, reamer planer 600, inserter/extractor handle assembly 700, height measurement gauge 800, impaction stand 900, and taper breaker tool 1000, for example.

In addition to that described above and illustrated in the figures, various other operations are now described with reference to FIGs. 19A-19M. It should be understood that the following operations do not have to be performed in the exact order described below. Instead, various operations may be handled in a different order or simultaneously. Operations may also be omitted or added unless otherwise stated therein.

In an exemplary method of replacing a proximal humerus 53, the proximal humerus 53 may be resected. This may be achieved by clamping bone clamp assembly 400 at a desired location along a length of humeral shaft 52, as shown in FIG. 19A. Bone clamp 402 may be adjusted until drop rod 440 extending through second jaw 411b is aligned parallel to a shaft axis of humeral shaft 52 which consequently aligns resection guide slot 424 and resection guide surface 424 perpendicular to humeral shaft 52. As mentioned above, teeth 415 of clamp jaws 411a-b may assist in aligning resection guide 420 perpendicular to the shaft axis. Once parallel alignment is achieved, knob 450 is tightened so as to secure clamp 402 to bone 52. It should be noted that resection guide 420 may be connected to bone clamp 402 in the manner previously described before or after bone clamp 402 is secured to bone 52.

Thereafter, a bone saw is guided along proximal guide surface 422 and through bone 52 to remove proximal portion 53 of humerus 50. Alternatively, the bone saw may be guided through captured guide slot 424 to resect proximal humerus 53. Once the proximal portion 53 of humerus 50 is resected, resection guide 420 may be disconnected from second jaw 411b while leaving bone clamp 402 connected to humeral shaft 52 such that a portion of the bone extends proximally from jaws 411a-b, as shown FIG. 19B. Clamp 402 may remain attached to bone 52 throughout the remainder of the procedure to help provide manual manipulation and control of the bone. Additional bone forming operations may then be performed. For example, cutting head 504 of sounder 500 may be inserted into an intramedullary canal 56 of shaft and used to shape intramedullary canal 56 for receipt of stem component 130. Subsequently or alternatively, reamer head 604 of reamer planar 600 may be inserted into the intramedullary canal 56 to further shape the intramedullary canal 56, while planer plate 601 may concurrently mill the proximal end of humeral shaft 52 so as to form a planar proximal surface.

Inserter/extractor handle assembly 700 may be connected to adapter 340 of expandable stem 330, as illustrated in FIGs. 15C-15E and FIG. 19C. In this regard, sleeve 730 of handle assembly 700 may be pulled proximally against the bias of spring 752 to expose arms 744. Arms 744 may then be slid into engagement with flats 349 on adapter 340 of expandable stem 330 and so that shoulders 341 are received in corresponding recesses 746. Sleeve 730 may then be released allowing the compression of spring 752 to advance sleeve 730 over adapter 740 to secured handle assembly 700 to expandable trial stem 330. Version rods 1100 may be connected to a proximal end of handle 710 and to adapter 340 of expandable stem 330, as shown in FIG. 19C.

Expandable trial stem 330 may then be inserted into bone 52 so that adapter 340 is positioned at the proximal end of the resected bone 52. Trial stem 330 may then be rotated within the bone until the desired version alignment is achieved. Once expandable trial 330 is inserted into bone 52 and the desired version is achieved, a driver 1200 may be inserted through handle assembly 700, into adapter 340, and into engagement with expansion bolt 335. Driver 1200 may then be rotated in a first direction thereby driving expansion bolt 335 in a distal direction which causes first and second stem portions 337a-b to move radially outwardly into engagement with the bone 52 to secure trial stem 330 thereto. Handle may then be removed by again pulling sleeve 730 proximally to the second position and sliding arms 744 away from flats 149.

In some embodiments, expandable trial stem 330 may not be used. Instead, implant stem component 130 may be implanted and then used for subsequent trialing operations. In such embodiments, inserter/extractor handle assembly 700 may be attached to adapter 140 of stem component 130 as described above with relation to expandable trial stem 330. However, prior to connecting handle assembly 700 to adapter 140 of stem component 130, taper protector insert 370 may be inserted into tapered bore 142 of adapter 140 such that post 378 extends proximally therefrom. Additionally, rather than placing stem 132 within the bone 52 as would be the case with first and second stem portions 337a-b prior to their expansion, stem component 130 may be rotated to the desired version position and impacted into the bone 52 using handle 710. Taper protector 370 may help protect tapered bore 142 and adapter 140 during impaction. Handle assembly 700 may then be removed from adapter 140 while leaving taper protector insert 370 within adapter 140.

Height gauge 800 may then be connected to adapter 340 of expandable stem trial 330 (or adapter 140 of implant stem component 130 if used instead). In this regard, mating member 811 may be positioned over adapter 340 and then pressed distally onto adapter 340 such that inner legs 813 snap into a locked arrangement with divots 346 and shoulders 347. Additionally, flats 348a-b on post 348 and within mating member 811 engage each other so that clamp 830 and pointer 820 are oriented toward the glenoid 58. Arm 834 of clamp 830 may then be depressed to slide clamp 830 superiorly or inferiorly along scale 810 until pointer tip 822 is positioned at a center of glenoid 58, as illustrated in FIG. 19E. Pointer 830 may also be slid medially toward glenoid 58 until it contacts the center point of glenoid 58. Arm 834 of clamp 830 may be released to secure clamp 830 in position on scale 810. A height measurement may then be read by referring to indicia 812 relative to pointer 820.

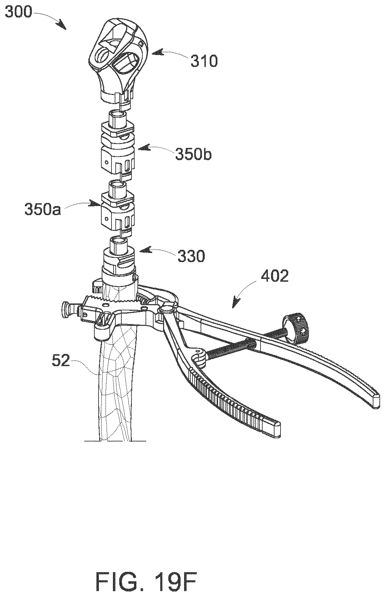

Based on the height measurement obtained via height gauge 800, one or more trial spacers 350 may be selected to match the measured height. For example, as shown in FIG. 19F, a first trial spacer 350a, which may correspond to a height of 20mm, and a second trial spacer 350b, which may correspond to a height of 30mm, for example, may be selected for a total height of 50mm. First trial spacer 350a may then be connected to adapter 340 by pushing spacer 350a onto adapter 340 until inner legs 352 thereof snap into place. Second trial spacer 350b may similarly be pushed onto first trial spacer 350a, and proximal trial body 310 may be pushed onto second trial spacer 350b, as shown in FIG. 19F. The corresponding flats 348a-b of adapter post 348 and flats 356a-b of spacer posts 356 ensures that trial proximal body 310 is properly oriented toward glenoid 58.

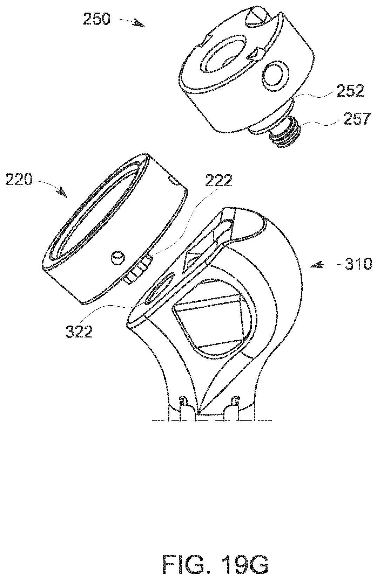

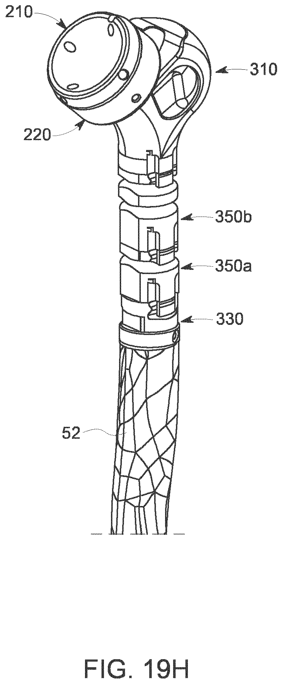

The appropriate trial joint component may then be assembled to trial proximal body 310. Some procedures may utilize a reverse joint construct in which the artificial joint is constructed reverse to that of the natural joint. In the natural joint, humeral head 51 is a convex structure which articulates with the concave glenoid 58. However, in a reverse procedure, the artificial construct is reversed such that the humeral articular component includes a concave articular surface and the glenoid includes a convex glenosphere for articulation with the concave articular surface of the humeral prosthesis. As illustrated in FIG. 19G, where a reverse joint construct is desirable, trial tray 220 may be coupled to proximal body 310 by inserting boss 222 into scalloped opening 321 within trial proximal body 310. A screw, such as screw 257, may be inserted through through-hole 229 in trial tray 220 and into engagement with threaded opening 325 in proximal body 310. Reverse insert 210 may then be connected to tray 220, as shown in FIG. 19H, and placed into engagement with a trial glenosphere to evaluate the functioning of the joint with the particular constructed arrangement. Initial trialing in this regard may be done with trial tray 220 in a first eccentricity configuration, such as that shown in FIG. 5E, which may correspond to a neutral eccentricity or no eccentricity. If it is determined that eccentricity is needed, tray 220 may be rotated so tray 220 is positioned in a second eccentricity configuration, such as that shown in FIG. 5F. Trialing of the joint is then repeated. Additional eccentricity may be applied until the desired eccentricity is achieved.

In procedures in which a standard or non-reverse artificial joint is desirable, humeral head coupling trial 250 and humeral head trial 230 may be connected to trial proximal body 310 in the same manner just described with relation to trial tray 220 and reverse insert 210. Trialing may also proceed in the same manner until the appropriate eccentricity is achieved. Also, should it be determined that the height of humeral trial prosthesis 300 is not sufficient, one or more trial spacers 350a-b may be swapped out for another trial spacer arrangement with a different total height.

After trialing with trial humeral prosthesis 300, humeral prosthesis 100 may be constructed to match the height and eccentricity determined through the trialing operations. In this regard, implant spacers, such as a first and second spacer 150a-b, may be selected to match the height of trial humeral prosthesis 300. Humeral prosthesis 100 may be assembled on a back-table using impaction stand 900, as described above.

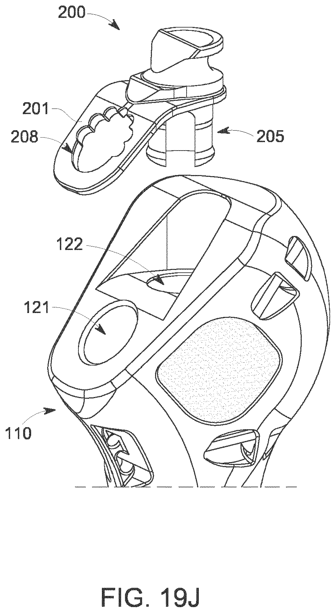

However, prior to assembling articular joint component 260 to implant proximal body 110. Additional trialing may be performed using humeral prosthesis 100. In this regard, connector 205 of proximal body adapter 200 may be inserted into second bore 122 of proximal body 110 so that flange 201 extends over first bore 121, as illustrated in FIGs. 3C and 19J. Trial tray 220 or humeral head coupler trial 250 may then be connected to proximal body 110 with the eccentricity determined from trialing with trial humeral prosthesis 300. The construct may then be trialed for functionality before final assembly. In some embodiments of the surgical procedure, trial humeral prosthesis 300 may not be utilized for trialing operations. Instead, humeral prosthesis 100 may instead be used for all trialing operations. The use of height gauge 800 minimizes the risk of an inappropriate prosthesis height requiring disassembly during such trialing operations, which may otherwise prohibit the use of humeral prosthesis 100 as a trial.

However, in some circumstances, it may be determined that humeral prosthesis 100 is either too short or too tall. Therefore, upon trialing humeral prosthesis 100, it may be desirable to remove one or more implant spacers 150a-b and/or replace one more implant spacers 150a-b with one or more implant spacers 150a-d of different sizes. However, this may prove difficult due to the taper locks between spacers 150a-b and each other and spacers 150a-b and stem component 130 and proximal body 110. Taper breaker tool 1000 may then be used which can make disconnecting a spacer 150 not only easy but also protective of the bone and stem interface. In this regard, taper breaker tool 1000 may be positioned along humeral prosthesis 100 such that first and second wedges 1008a-b are aligned with a desired interface. Such interface may be between adjacent implant spacers 150, between a spacer 150 and adapter 140 of stem component 130, or between a spacer 150 and implant proximal body 110. Drive member 1005 may then be driven to push moveable block 1006 toward first wedge 1008a resulting in separation of the desired components.

Once trialing is sufficiently complete, joint component 260 may be assembled to proximal body 110 and soft tissue may be sutured to humeral prosthesis 100. In this regard, some soft tissue structures, such as some or all of the muscles of the rotator cuff, for example, may be attached to implant proximal body 110 via sutures such that soft tissue compressed against porous patches 117. Other soft tissue structures, such as the deltoid and some of the rotator cuff muscles, for example, may be attached to one or more of implant spacers 150a-b so that tissue is compressed against porous rings 156.

The aforementioned devices, systems, and methods have been described in the context of manually performed humeral replacement procedures. However, in some embodiments, replacement of proximal humerus 53 with humeral prosthesis 100 may also be performed robotically or performed with the aid of a computer assisted surgery ("CAS") system.

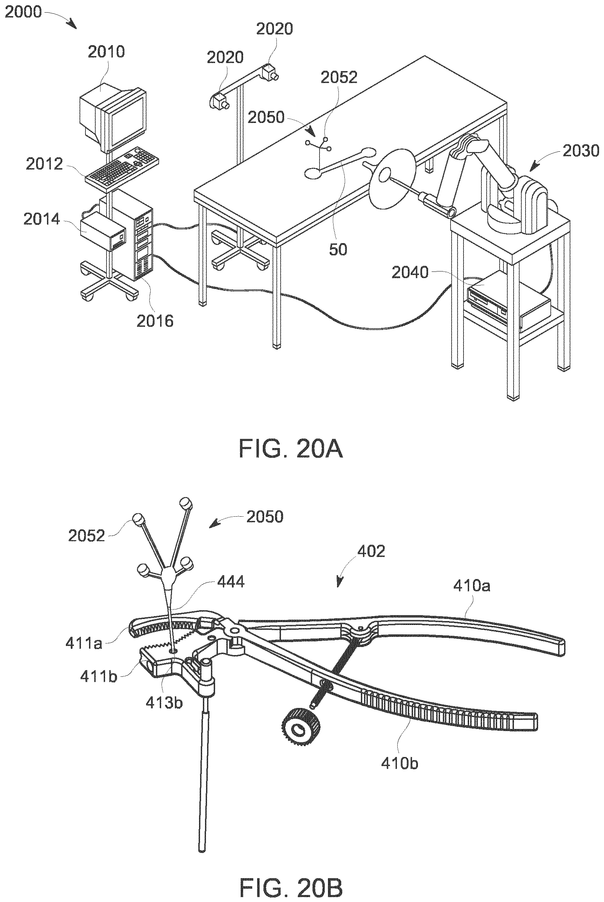

FIG. 20A is a diagrammatic illustration of an exemplary CAS system 2000. In this regard, CAS system 2000 may include a haptic device 2030 to help guide the surgeon in performing various operations of the procedure, such as bone cutting. However, it should be understood that system 2000 may also be utilized in a robotic procedure in which, instead of haptic device 2030, an autonomous or semi-autonomous robot is utilized to perform such operations. CAS system 2000 may also include a display device 2010, an input device 2012, and a processor-based system 2016, which may include, for example, a computer 2040 with a processor and storage medium 2014, and a visual tracking system that may include one or more cameras 2020, which may be communicatively connected to processor-based system 2016. One example of a CAS system that may be utilized to replace proximal humerus is described in greater detail in U.S. Patent No. 7,831,292, the disclosure of which is hereby incorporated by reference herein.

A surgical procedure for replacing a proximal humerus utilizing CAS system 2000 may begin by registering the patient's target anatomy (*i.e.,* humerus 50) with the system 2000. This may be performed by connecting an anatomy tracker 2050 to humerus 50. Anatomy tracker may include a marker array that may include a plurality of markers 2052 positioned in a predetermined arrangement. Markers 2052 may each be an active marker or a passive marker. An example of an active marker is a light emitting diode ("LED"). An example of a passive marker is a reflective marker, such as ball-shaped marker with a surface that reflects incident infrared radiation. Regardless of the markers 2052 utilized, cameras 2020 may be configured to detect and track markers 2052 which communicate the locations of such markers 2052 in an operative coordinate space to the processor-based system 2016 so that the processor thereof can map such locations to a virtual coordinate space. This allows operations to be performed on the anatomy via haptic device 2030, or an autonomous or semi-autonomous robot, in accordance with a preoperative plan.

Once anatomy tracker 2050 is connected to the bone 50, a probe (not shown) also with a marker array may be used to contact various points on humerus 50. Processor-based system 2016 correlates the probe locations with the location of anatomy tracker 2050 on bone 50 so that the specific anatomy of humerus 50 can be mapped into the virtual coordinate system. Thus, with registration of humerus 50 completed, various surgical operations may be performed on humerus 50, such as resecting proximal humerus 53 along humeral shaft 52 and reaming an intramedullary canal of humeral shaft 52.

In some methods of performing a robotic or CAS procedure, anatomy tracker 2050 may not be connected to bone 50, but may instead be connected to bone clamp 402, as illustrated in FIG. 20B. As shown in FIG. 20B, anatomy tracker 2050 may be connected to second jaw 411b of bone clamp, such as to second hole 413b. However, in other embodiments anatomy tracker 2050 may be connected to bone clamp 402 at another location on second jaw 411b or on one of first and second handles 410a-b, for example. Anatomy tracker may remain connected to bone clamp 402 during the procedure or may be removed and connected as necessary during various stages of the procedure. In any event, registration of humerus 50 may be performed using anatomy tracker 2050 while tracker 2050 is connected to bone clamp 402, and bone clamp 402 is clamped to humerus 50.

After humerus is registered with CAS system 2000, various operations may be performed on the bone using CAS system 2000. For example, haptic device 2030 may be used to guide a cutting tool, such as a rotating burr, bone saw and the like, connected thereto through humeral shaft 52 proximal to jaws 411a-b of bone clamp 402. Similarly, where an autonomous or semi-autonomous robot is utilized, such robot may perform the cutting operations to remove proximal humerus 53. Still further operations may be performed using haptic device 2030 or a robot while bone clamp 402 and anatomy tracker 2050 remain connected to the bone. Such operations can include reaming an intramedullary canal of the bone, for example.

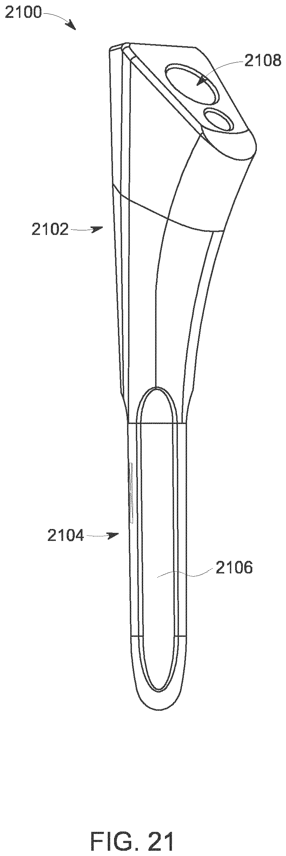

FIG. 21 depicts an exemplary humeral prosthesis 2100 according to another embodiment of the present disclosure. Humeral prosthesis 2100, unlike humeral prosthesis 100, is configured for implantation into humerus 50, such as in a primary or revision surgery in which there exists enough bone stock to encapsulate prosthesis 2100, as described above with respect to FIG. 9E. In this regard, humeral prosthesis 2100 may include a metaphyseal portion 2102 and a stem 2104 extending from metaphyseal portion 2102. Stem 2104 may be configured to be disposed within an intramedullary canal of humerus 50 while metaphyseal portion 2102 may be configured to be disposed within a metaphysis of a humerus 50 such that metaphyseal portion 2102 is recessed within or substantially flush with a proximal end of humerus 50. Humeral prosthesis 2100 may be configured for cemented implantation, such that stem 2104 may have a plurality of flutes 2106 configured to receive a sufficiently thick cement mantle to secure stem 2104 to bone 50, for example. In other embodiments, humeral prosthesis 2100 may be configured to be press-fit within humerus 50 and may include a porous exterior at least along a portion of metaphyseal portion 2102 to promote bone ongrowth/ingrowth. Metaphyseal portion 2102 may also include a bore 2108, which may be like bore 121, such that it may be configured to receive any of the final implant and trial components previously described with respect to FIGs. 3A-8B. Also, as described below, bore 2108 may also be configured to receive a modular adapter.

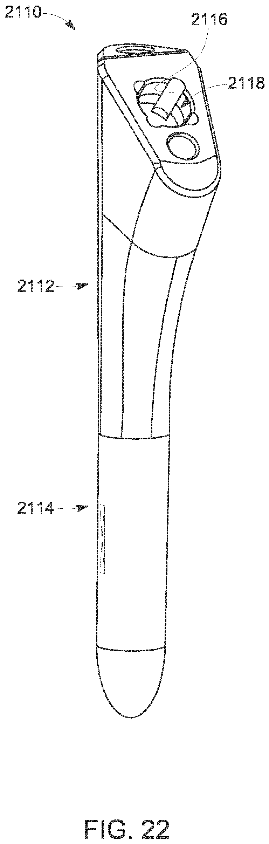

FIG. 22 depicts an exemplary humeral trial prosthesis 2110 according to another embodiment of the present disclosure. Humeral trial prosthesis or humeral broach 2110 is a trial prosthesis corresponding to humeral prosthesis 2100. As such, humeral trial prosthesis 2110 may have a similar geometry to that of humeral prosthesis 2100 and may include a metaphyseal portion 2112 and stem 2114. Also, similar to humeral prosthesis 2100, humeral trial 2110 may also include a bore 2118, which may be a scalloped bore like bore 321, or may be a smooth bore like bore 121. Stem 2114 may be an expandable stem like expandable trial stem 332. However, in other embodiments humeral trial prosthesis 2110 may be configured as a broach such that stem 2114 and/or metaphyseal portion 2112 may include cutting teeth or broach surfaces configured to remove bone from humerus 50 to conform to the exterior geometry of trial prosthesis 2110. When used as a broach, humeral trial prosthesis 2110 may be left within humerus 50 after broaching to conduct a trialing procedure, as discussed further below.

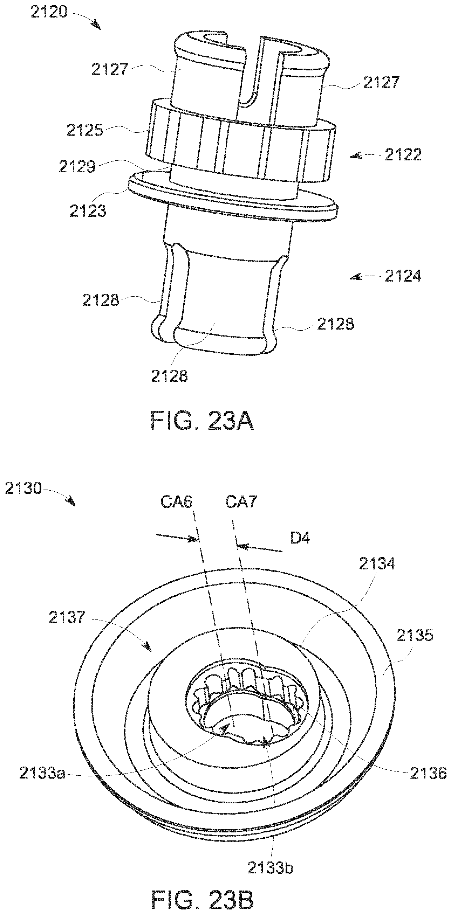

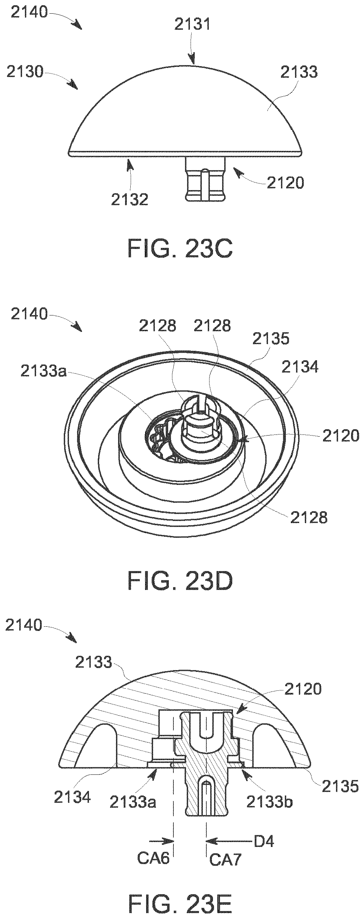

FIG. 23A depicts a modular adapter 2120 according to an embodiment of the present disclosure. Modular adapter or plug 2120 may include a proximal portion 2112, a distal portion 2124, and an annular flange or disc 2123 disposed between proximal and distal portions 2122, 2124. Proximal portion or first portion 2122 may include a splined section that may include a plurality of splines 2125 arrayed circumferentially about a longitudinal axis of modular adapter 2120 such that each spline 2125 extends in a proximal-distal direction. Proximal portion 2122 may also include a connection feature which, as shown, may include a plurality of flexures or flexible arms 2127 that extend proximally from the splined section and may be moveable radially inwardly from a first position to a second position. Proximal portion 2122 may include two or more flexures 2127, such as two flexures 2127, for example. A circumferential groove 2129 may be disposed between annular flange 2123 and the splined section to facilitate engagement of a removal tool (not shown). Distal portion or second portion 2124 may extend distally from annular flange 2123 and may also include a connection feature that may include a plurality of distally extending flexures or flexible arms 2128. As an example, distal portion 2124 may include two or more flexures 2128, such as three flexures 2128, for example. Flexures 2127, 2128 of proximal and distal portions 2122, 2124 provide for a snap-fit connection with a humeral head trial (described below) and bore 2108, 2118 of either humeral prosthesis 2100 or humeral trial prosthesis 2110. However, other connection mechanisms are contemplated, such as a threaded connection or taper-fit connection, for example.

Modular adapter 2120 may be made with a radiopaque material. For example, modular adapter 2120 may be made with a biocompatible polymer, such as Radel R5000, for example, with a radiopaque material added to the polymer composition. Such radiopaque material may include radium sulfate, for example. This may facilitate visualization of modular adapter 2120 via fluoroscopy, or other medical imaging, during a surgical procedure to assesses an eccentric axis of a humeral head trial during a procedure and may also help locate the modular adapter 2120 within the patient's surgical wound, should it inadvertently be left within the patient.

FIG. 23B depicts a humeral head trial 2130 according to another embodiment of the present disclosure. Humeral head trial 2130 may include an articular side 2131 and a connection side 2132. Articular side 2131 may include a convex articular surface 2133. Connection side 2132 may include a cavity 2137 extending therein and may define a circumferential rim 2135 that defines a periphery of humeral head trial 2130. A boss or central body 2134 may be disposed within cavity 2137. Body 2134 may include first and second openings 2133a-b extending therein. As shown in FIG. 23B, first opening 2133a may define a central axis CA6, and second opening 2133b may define a central axis CA7. Central axes CA6 and CA7 may be parallel and offset from each other by a distance D4. Distance D4 at least partially determines a potential offset of humeral head trial 2130 which may be 1mm to 4mm, for example. Central axis CA6 of first opening 2133a may be coaxial with a central axis of modular humeral head trial 2130 such that axis CA6 substantially intersects an apex or center of articular surface 2133. First and second openings 2133a-b may intersect each other so as to form a larger, oblong opening. In other words, first and second openings 2133a-b may each define a radius, and the distance D4 may be less than the sum of the radii of first and second openings 2133a-b. First and second openings 2133a-b may each include splines or scallops 2136 arrayed about their respective perimeters. In this regard, each of first and second openings 2133a-b may be configured to respectively receive proximal portion 2122 of modular adapter 2120 such that the splines 2125 thereof index with splines 2136 of first and second openings 2133a-b when separately disposed therein.

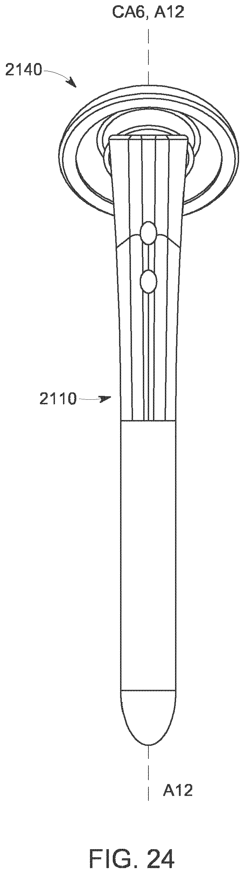

FIGs. 23C-23E illustrate a modular humeral head assembly 2140 that may include humeral head trial 2130 and modular adapter 2120. In this regard, modular adapter 2120 may be connected to modular head trial 2130 in either a first or second eccentricity configuration. In the first eccentricity configuration, proximal portion 2122 of modular adapter 2120 may be received within first opening 2133a of humeral head trial 2130 such that splines 2125 are indexed with splines 2136 of first opening 2133a to prohibit relative rotation and the longitudinal axis of modular adapter 2120 is coaxial with central axis CA6. Flexures 2127 of proximal portion 2122 create a quick-connect/disconnect mechanism that secures modular adapter 2120 to humeral head trial 2130 while allowing for its disconnection during a trialing operation. The first eccentricity configuration may be a neutral eccentricity. This neutral eccentricity configuration is illustrated in FIG. 24 in which distal portion 2124 of modular adapter 2120 is received within bore 2118 of trial prosthesis 2110 and central axis CA6 and a longitudinal axis A12 of humeral trial prosthesis 2110 are coplanar. Thus, humeral head trial assembly 2140 is centered in an A-P direction relative to trial prosthesis 2110.

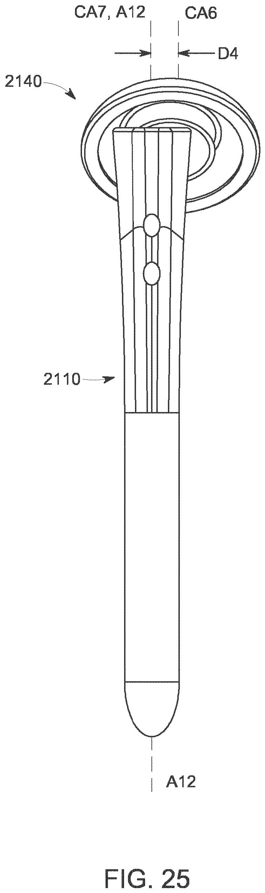

In the second eccentricity configuration, proximal portion 2122 of modular adapter 2120 may be received within second opening 2133b of humeral head trial 2130 such that splines 2125 are indexed with splines 2136 of second opening 2133b and the longitudinal axis of modular adapter 2120 is coaxial with central axis CA7 of second opening 2133b. Again, flexures 2127 facilitate a quick-connect/disconnect mechanism that secures modular adapter 2120 to humeral head trial 2130. The second eccentricity configuration may define an anterior or posterior eccentricity with a magnitude of D4. This arrangement is illustrated in FIG. 25 in which distal portion 2124 of modular adapter 2120 is received within bore 2118 of trial prosthesis 2110 and longitudinal axis A12 of humeral trial prosthesis 2110 is coplanar with central axis CA7. As shown, central axis CA6 is offset in the anterior or posterior direction by the distance D4. It should be noted that distal portion 2124 of modular adapter 2120 may be indexed to bore 2118 of trial prosthesis 2110 to provide a plurality of orientations such that modular head assembly 2140 can provide anterior eccentricity and posterior eccentricity depending on modular adapter's orientation within bore 2118. This may be done via splines or scallops 2116 within bore 2118 and corresponding splines on distal portion 2124.

In use, humeral trial prosthesis 2110 may be inserted into a proximal end of humerus 50 to form a bone void using the broach surfaces thereof. Trial 2110 may be left within the bone, and humeral head trial 2130 may be assembled with modular adapter 2120 in either the first or second eccentricity configuration. Humeral head trial assembly 2140 may then be connected to bore 2118 of trial prosthesis 2110 via flexures 2128 of distal portion 2124 of modular adapter 2120. Trialing with a glenoid component may then be performed. Should a different eccentricity ultimately be desired, humeral head trial assembly 2140 may be removed from humeral trial prosthesis 2110 and modular adapter 2120 moved to another of the first and second openings 2133a-b to provide for a different eccentricity configuration. Alternatively, another humeral head trial 2130 with a different D4 offset dimension may be utilized, as needed. Thus, a kit can be provided with a plurality of humeral head trials 2130 with different offsets to accommodate different eccentricity configurations. Humeral head trial 2130 may then be reconnected to trial prosthesis 2110 and trialing performed again. Once humeral prosthesis 2100 is implanted after this initial trialing, a secondary trialing may again be performed using humeral prosthesis 2100 and humeral head trial assembly 2140 in the same manner.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A system for replacing a proximal humerus, comprising:
a prosthesis body having a first superior surface, an anterior surface, a posterior surface, a lateral surface, a medial surface, and a first bore extending through the first superior surface, the first bore having a plurality of scallops arrayed about a central axis of the first bore; and
an articular component having a component body and a boss extending from the component body, the component body having an articular surface disposed thereon, the boss being configured to be received within the first bore and having a protrusion extending radially outwardly therefrom and being configured to engage one of the scallops.

2. The system of claim 1, wherein:
the component body includes a central axis and the boss defines a boss axis, the central axis being offset from the central axis, and
the prosthesis body includes a midline plane bisecting the prosthesis body and extending in a superior-inferior direction and a medial-lateral direction.

3. The system of claim 2, wherein the articular component has a first eccentricity configuration relative to the prosthesis body in which the protrusion engages a first scallop and the central axis of the component body and the boss axis are coplanar with the midline plane.

4. The system of claim 3, wherein the articular component has a second eccentricity configuration relative to the prosthesis body in which the protrusion engages a second scallop, the boss axis is coplanar with the midline plane, and the central axis of the component body is offset in one of an anterior and posterior direction relative to the midline plane.

5. The system of as in any of claims 2-4, wherein the component body includes a central opening coaxial with the central axis of the body and a through-bore extending through the component body and the boss, the through-bore being coaxial with the boss axis.

6. The system as in any of the preceding claims, wherein the articular surface is a concave articular surface.

7. The system as in any of the preceding claims, wherein the articular surface is a convex articular surface.

8. The system as in any of claims 2-4, wherein articular component includes a reverse trial insert having a concave articular surface, and the component body is a tray having an inner surface bounded by a rim such that the rim and the inner surface together define a cavity configured to receive the reverse trial insert.

9. The system as in any of claims 2-4, wherein:
the articular component includes a humeral head trial having a convex articular surface and a socket disposed opposite the convex articular surface, and
the component body is configured to be received within the socket of the humeral head trial.

10. The system of claim 9, wherein the component body includes an axial slot, and the articular component includes a rib disposed within the socket, the rib being configured to be received within the axial slot of the component body.

11. The system as in any of the preceding claims, wherein the prosthesis body includes a second bore extending entirely therethrough.

12. The system of claim 11, further comprising a trialing adapter having a connection portion receivable within the second bore and a flange extending from the connection portion, the flange having an opening extending therethrough, the opening at least partially defining the first bore of the prosthesis body when the connection portion is received within the second bore, and wherein the scallops are disposed within the flange about the opening.

13. The system as in any of the preceding claims, wherein the lateral surface of the prosthesis body includes a spherical curvature, and the anterior and posterior surfaces are each planar and each intersect the first superior surface, the lateral surface, and the medial surface.

14. The system as in any of the preceding claims, wherein the prosthesis body includes a first suture opening and a second suture opening, the first suture opening defines a first suture opening axis, the second suture opening defines a second suture opening axis, and the first and second axes intersect at an angle O.

15. The system of claim 14, wherein the anterior surface includes a porous patch and at least one of the first suture opening axis and second suture opening axis overlaps the porous patch.
